# EUROPEAN PATENT SPECIFICATION

(11) **EP 2 791 324 B1**
(45) Date of publication and mention of the grant of the patent: **10.05.2017**
(21) Application number: 12856927.4
(22) Date of filing: 13.12.2012
(51) Int. Cl.: C12N 5/0793, C12N 5/0797, A61K 45/00, A61P 25/18, A61P 25/28

(54) **CELL POPULATION FOR USE IN TREATING NEUROPSYCHIATRIC DISORDERS**
ZELLPOPULATION ZUR VERWENDUNG IN DER BEHANDLUNG VON NEUROPSYCHIATRISCHEN ERKRANKUNGEN.
POPULATION DE CELLULES UTILISÉES POUR LE TRAITEMENT DE TROUBLES NEUROPSYCHIATRIQUES

(30) Priority: 13.12.2011 AU 2011905180
(43) Date of publication of application: 22.10.2014
(73) Proprietor: Medvet Science Pty Ltd, Underdale, SA 5032 (AU)
(72) Inventor: LOPEZ, Angel, Frome Road Adelaide SA 500 (AU); SCHWARZ, Quenten, Frome Road Adelaide SA 500 (AU)
(74) Representative: D Young & Co LLP
(86) International application number: PCT/AU2012/001530
(87) International publication number: WO 2013/086574

(56) References cited:
- WO-A1-2013/040650
- WO-A2-00/52143
- WO-A2-2011/096728
- Q. SCHWARZ ET AL: "Neuropilin-mediated neural crest cell guidance is essential to organise sensory neurons into segmented dorsal root ganglia", DEVELOPMENT, vol. 136, no. 11, 1 June 2009 (2009-06-01) , pages 1785-1789, XP055177473, ISSN: 0950-1991, DOI: 10.1242/dev.034322
- Q. SCHWARZ ET AL: "Neuropilin 1 and 2 control cranial gangliogenesis and axon guidance through neural crest cells", DEVELOPMENT, vol. 135, no. 9, 26 March 2008 (2008-03-26), pages 1605-1613, XP055177487, ISSN: 0950-1991, DOI: 10.1242/dev.015412
- P-S CHEAH ET AL: "Neurodevelopmental and neuropsychiatric behaviour defects arise from 14-3-3[zeta] deficiency", MOLECULAR PSYCHIATRY, vol. 17, no. 4, 29 November 2011 (2011-11-29), pages 451-466, XP55147333, ISSN: 1359-4184, DOI: 10.1038/mp.2011.158
- EL SEADY, R. ET AL.: 'Uncomplicated differentiation of stem cells into bipolar neurons and myelinating glia' BIOCHEMICAL AND BIOPHYSICAL RESEARCH COMMUNICATIONS vol. 376, 2008, pages 358 - 362, XP025472405
- HSIEN-CHENG HUANG, A. ET AL.: 'Putative Dental Pulp-Derived Stem/Stromal Cells Promote Proliferation and Differentiation of Endogenous Neural Cells in the Hippocampus of Mice' STEM CELLS vol. 26, 2008, pages 2654 - 2663, XP055071928
- WRIGHT, I.C. ET AL.: 'Meta-Analysis of Regional Brain Volumes in Schizophrenia' AM J PSYCHIATRY vol. 157, no. 1, 2000, pages 16 - 25, XP055071931
- KEMPTON, M.J. ET AL.: 'Structural Neuroimaging Studies in Major Depressive Disorder' ARCH GEN PSYCHIATRY vol. 68, no. 7, July 2011, pages 675 - 690, XP003031514
- GAMMILL, L.S. ET AL.: 'Guidance of trunk neural crest migration requires neuropilin 2/semaphorin 3F signaling' DEVELOPMENT vol. 133, no. 1, 2006, pages 99 - 106, XP055071934
- CHEN, H. ET AL.: 'Neuropilin-2 Regulates the Development of Select Cranial and Sensory Nerves and Hippocampal Mossy fiber Projections' NEURON vol. 25, 2000, pages 43 - 56, XP003031515

## Description

### FIELD OF THE INVENTION

The present invention relates generally to the regeneration of the hippocampus in a mammal and agents for use therein. More particularly, the present disclosure provides a method of regenerating the hippocampus in a mammal by administering a sub-population of neural crest stem cells. The method of the present disclosure is useful in the treatment of conditions characterised by a defective hippocampus, such as neuropsychiatric disorders.

### BACKGROUND OF THE INVENTION

Bibliographic details of the publications referred to by author in this specification are collected alphabetically at the end of the description.

The reference to any prior art in this specification is not, and should not be taken as, an acknowledgment or any form of suggestion that that prior art forms part of the common general knowledge in Australia.

Schizophrenia is one of the most disabling and emotionally devastating illnesses known to man. Unfortunately, because it has been misunderstood for so long, it has received relatively little attention and its victims have been undeservingly stigmatized. Schizophrenia is in fact, a fairly common disorder. It affects both sexes equally and strikes about 1% of the population worldwide. Another 2-3% have *schizotypal personality disorder,* a milder form of the disease. Because of its prevalence and severity, schizophrenia has been studied extensively in an effort to develop better criteria for diagnosing the illness.

Schizophrenia is characterized by a constellation of distinctive and predictable symptoms. The symptoms that are most commonly associated with the disease are called positive symptoms, that denote the presence of grossly abnormal behaviour. These include thought disorder (speech which is difficult to follow or jumping from one subject to another with no logical connection), delusions (false beliefs of persecution, guilt, grandeur or being under outside control) and hallucinations (visual or auditory). Thought disorder is the diminished ability to think clearly and logically. Often it is manifested by disconnected and nonsensical language that renders the person with schizophrenia incapable of participating in conversation, contributing to his alienation from his family, friends and society. Delusions are common among individuals with schizophrenia. An affected person may believe that he is being conspired against (called "paranoid delusion"). "broadcasting" describes a type of delusion in which the individual with this illness believes that his thoughts can be heard by others. Hallucinations can be heard, seen or even felt. Most often they take the form of voices heard only by the afflicted person. Such voices may describe the person's actions, warn him of danger or tell him what to do. At times the individual may hear several voices carrying on a conversation. Less obvious than the above "positive symptoms" and "thought disorder" but equally serious are the deficit or negative symptoms that represent the absence of normal behaviour. These include flat or blunted affect (i.e. lack of emotional expression), apathy, social withdrawal and lack of insight.

The onset of schizophrenia usually occurs during adolescence or early adulthood, although it has been known to develop in older people. Onset may be rapid with acute symptoms developing over several weeks, or it may be slow developing over months or even years. While schizophrenia can affect anyone at any point in life, it is somewhat more common in those persons who are genetically predisposed to the disease with the first psychotic episode generally occurring in late adolescence or early adulthood. The probability of developing schizophrenia as the offspring of two parents, neither of whom has the disease, is 1 percent. The probability of developing schizophrenia as the offspring of one parent with the disease is approximately 13 percent. The probability of developing schizophrenia as the offspring of both parents with the disease is approximately 35 percent. This is indicative of the existence of a genetic link.

Three-quarters of persons with schizophrenia develop the disease between 16 and 25 years of age. Onset is uncommon after age 30 and rare after age 40. In the 16-25 year old age group, schizophrenia affects more men than women. In the 25-30 year old group, the incident is higher in women than in men.

In general, the study of any illness requires that there should be good criteria for diagnosis. In fact, diagnosis should ultimately be based on causes i.e., on whether an illness results from a genetic defect, a viral or bacterial infection, toxins or stress. Unfortunately, the causes of most psychiatric illnesses are unknown and therefore these disorders are still grouped according to which of the four major mental faculties are affected:
(i) disorders of thinking and cognition
(ii) disorders of mood
(iii) disorders of social behaviour; and
(iv) disorders of learning, memory and intelligence.

Accordingly, since so little is known of the biological causes of these conditions, there is an ongoing need to elucidate the mechanisms by which these diseases are induced and progress.

The 14-3-3 proteins constitute a family of highly conserved regulatory molecules expressed abundantly throughout development and in adult tissue. These proteins comprise seven distinct isoforms (β, ζ, ε, γ, η, τ, σ), that bind a multitude of functionally diverse signalling molecules to control cell cycle regulation, proliferation, migration, differentiation and apoptosis (Berg et al. Nat Rev Neurosci 2003; 4(9):752-762; Fu et al. Annu Rev Pharmacol Toxicol 2000; 40:617-647; Toyo-oka et al. Nat Genet 2003 Jul; 34(3): 274-285; Aitken A., Semin Cancer Biol 2006; 16(3):162-172; Rosner et al. Amino Acids 2006; 30(1):105-109).

To date, the role, if any, of the protein 14-3-3 family of molecules in schizophrenia has remained elusive. Some research has focussed, albeit so far inconclusively, on identifying single nuclear polymorphisms associated with a predisposition to developing a neuropsychiatric condition such as schizophrenia. Studies aimed at investigating changes to levels of protein 14-3-3 isoforms, irrespective of whether or not those molecules are mutated, have tended to focus on changes to the levels of the eta and theta isoforms, although to date there has not been any conclusive evidence that they are reliable markers of the onset of a neuropsychiatric condition. In relation to other of the protein 14-3-3 isoforms, such as beta and zeta. Wong *et al.* (2005) found no change to expression levels in schizophrenia and bipolar disorders. Middleton *et al.* (2005) went further and stated that these particular isoforms are not likely to be directly related to a genetic risk for developing schizophrenia and that neither marker provides a strong association with schizophrenia.

Nevertheless, and contrary to these findings, in work leading up to the present invention it has been determined that a reduction in the functional level of protein 14-3-3ζ, in particular the level of 14-3-3ζ/DISCl formation, is associated with the onset of or predisposition to the onset of a neuropsychiatric disorder, such as a condition which is characterised by one or more symptoms of schizophrenia.

Although these findings are certainly highly relevant in terms of the development of a diagnostic for predicting the susceptibility to the onset of schizophrenia, the person of skill in the art would appreciate that the existence of a diagnostic symptom does not necessarily teach towards a potential therapy since detectable diagnostic markers, although reliable as a marker, *per se,* are often secondary to the actual cause of the disease. Without direct knowledge of "cause and effect" in relation to a disease condition, the design of an effective therapeutic is rendered virtually impossible. To this end, the development of a method of effectively treating a neuropsychiatric disorder, such as schizophrenia, has been long sought after.

To this end, in further work leading up to the present invention the defect in 14-3-3ζ and 14-3-3ζ/DISCl complex functionality has been determined to lead.to developmental abnormalities of the hippocampus arising from aberrant neuronal migration. Still further, in terms of the development of the hippocampus it has been determined that the Nrp2⁺ neural crest stem cells, being a subpopulation of neural crest stem cells, specifically differentiate to neurons of the hippocampus and can effectively regenerate the hippocampus. This has therefore now facilitated the design of a therapeutic treatment for neuropsychiatric conditions, such as schizophrenia.

### SUMMARY OF THE INVENTION

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integers or steps.

As used herein, the term "derived from" shall be taken to indicate that a particular integer or group of integers has originated from the species specified, but has not necessarily been obtained directly from the specified source. Further, as used herein the singular forms of "a", "and" and "the" include plural referents unless the context clearly dictates otherwise.

Unless otherwise defined, all technical and scientific terms used herein have the same meaning as commonly understood by one of ordinary skill in the art to which this invention belongs.
The present invention provides an Nrp2⁺ neural crest stem cell population or variants thereof for use in the treatment of a neuropsychiatric condition in a mammal, as specified in the claims.

One aspect of the present disclosure is directed to a method of treating a mammal with a condition characterised by a defective hippocampus, said method comprising administering to said mammal an effective number of Nrp2⁺ neural crest stem cells or mutants or variants thereof for a time and under conditions sufficient to effect regeneration of the hippocampus.

In another aspect there is provided a method of treating a human with a condition characterised by a defective hippocampus, said method comprising administering to said mammal an effective number of Nrp2⁺ neural crest stem cells or mutants or variants thereof for a time and under conditions sufficient to effect regeneration of the hippocampus.

In still another aspect, there is therefore provided a method of treating a mammal with a condition characterised by a defective hippocampus, said method comprising administering to said mammal an effective number of adult Nrp2⁺ neural crest stem cells or mutants or variants thereof for a time and under conditions sufficient to effect regeneration of the hippocampus.

Yet another aspect of the present disclosure is directed to the use of Nrp2⁺ neural crest stem cells or mutants or variants thereof in the manufacture of a medicament for the treatment of a condition in a mammal, which condition is characterised by a defective hippocampus, wherein said stem cells regenerate the hippocampus.

A further aspect of the present disclosure isdirected to an isolated cellular population comprising Nrp2⁺ neural crest stem cells for use in the method of the disclosure.

### BRIEF DESCRIPTION OF THE DRAWINGS

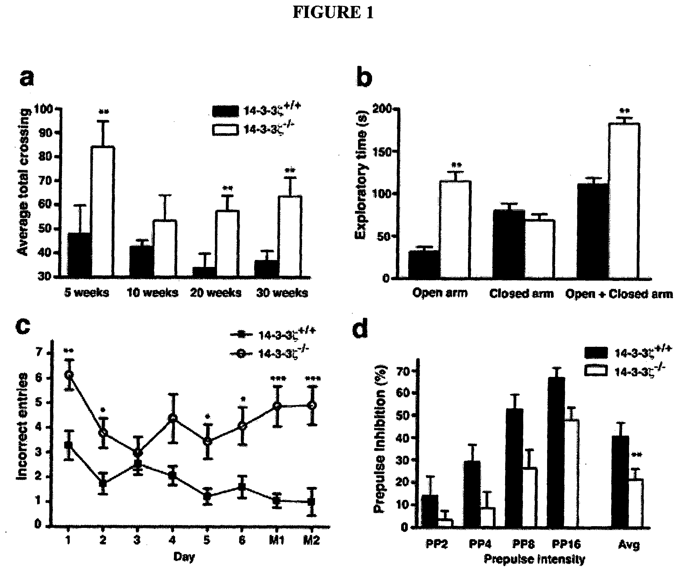

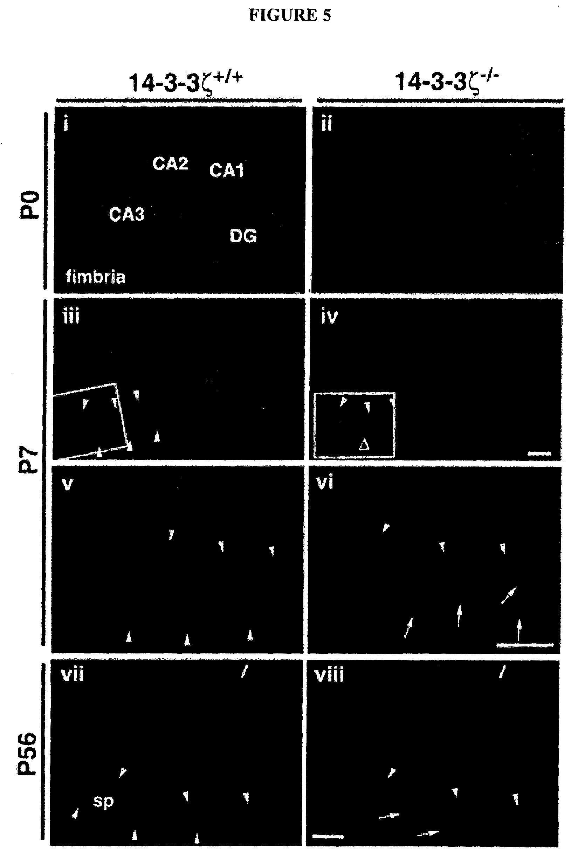

**Figure 1****: 14-3-3ζ-deficient mice demonstrate abnormal cognitive and behavioural traits.**
   14-3-3ζ^{062-/-} mice (open bars: n=11) have greater exploratory behaviour at 5-30 weeks of age than 14-3-3ζ^{062+/+} littermates (filled bars; n=11) in an open field test. (b) 14-3-3ζ^{062-/-} mice (open bars; n=12) spend more time than 14-3-3ζ^{062+/+} mice (filled bars; n=12) in the open arm in an elevated plus maze. (c) 14-3-3ζ^{062-/-} mice (open circles; n=12) have lower capacity than 14-3-3ζ^{062+/+} mice (closed squares; n=12) for both spatial learning (Day 1-6) and memory (M1 and M2) in a cross maze escape task test. (d) Compared to 14-3-3ζ^{062+/+} mice (filled bars; n=11) the 14-3-3ζ^{062-/-} mice (open bars; n=11) have reduced PPI with a prepulse (PP) of 2, 4, 8 and 16 dB over the 70 dB baseline and an inter-stimulus interval of 100 msec. The average (Avg) of data from all PP intensities is also shown. Data from male and female mice is pooled in all graphs. Error bars are presented as mean ± SEM. *, *p*< 0.05; ***, p* < 0.01; ***, *p*< 0.001.

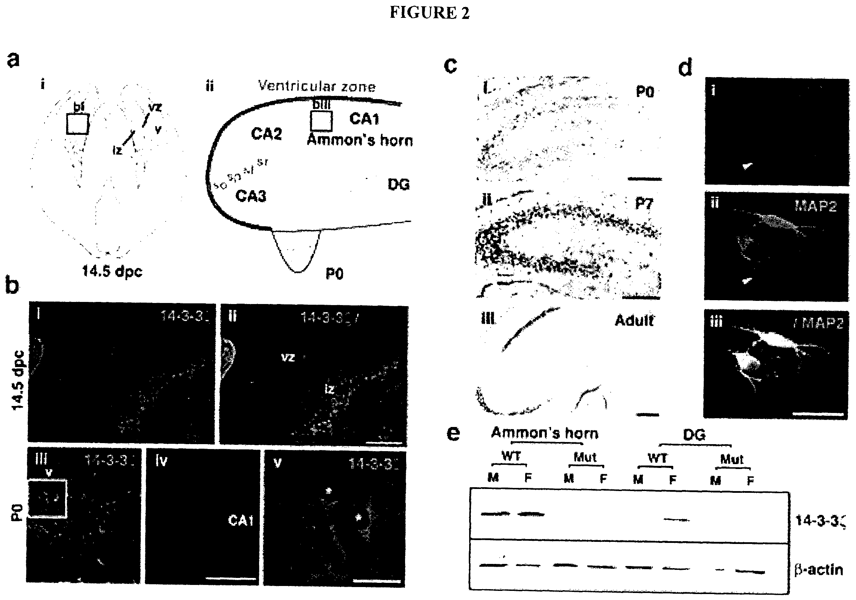

**Figure 2****. 14-3-3ζ is expressed in the pyramidal cells of Ammon's horn and granule neurons of the dentate gyrus.**(a) (i) Schematic representation of a coronal section through a 14.5 dpc embryonic mouse brain depicting the different regions of the hippocampus, V, ventricle; IZ, intermediate zone; VZ. ventricular zone. (ii) Schematic representation of a coronal section through P0 mouse hippocampus. Neurons from the hippocampal primordium originate from the ventricular neuroepithelium (light blue) and neuroepithelium adjacent to the fimbria (dark blue). The three subfields containing the pyramidal neurons of the cornu ammonis (CA1-3) that compose Ammon's horn and its layers (so, stratum oriens; sp, stratum pyramidale; sl, stratum lucidum; sr, stratum radiatum) are depicted in relation to positioning of granular neurons in the dentate gyrus (DG). (b) (i-ii) 14-3-3ζ immunoreactivity was detected in the intermediate zone of the 14.5 dpc developing hippocampus. (iii-iv) At P0, 14-3-3ζ-positive neurons are located in the pyramidal cell layer. (v) Higher magnification of the pyramidal neurons (asterisks) shows that 14-3-3ζ has a punctate cytoplasmic localisation. (c) X-gal staining showing the endogenous expression of *14-3-3ζ* in P0, P7 and adult 14-3-3ζ^{062+/-} hippocampi. The high level of 14-3-3ζ-lacZ expression is evident in pyramidal and granular neurons. (d) Hippocampal neuronal culture. (i) 14-3-3ζ staining with EB1 (red). (ii) MAP2 positive (green) hippocampal neurons. (iii) Overlay of 14-3-3ζ and MAP2 highlights the co-expression in MAP2 positive neurites (arrow). (e) 14-3-3ζ protein (27 kDa) is expressed in Ammon's horn and dentate gyrus of the WT mice. Western blot of lysates from adult WT and 14-3-3ζ^{062-/-}. mice were immunoblotted and probed with antibody to 14-3-3ζ(EB1). Anti-(3-actin (42 kDa) antibody was used as a loading control. Scale bars = 100 µm (bi-iv; c: di-iii), 25 µm (bv).

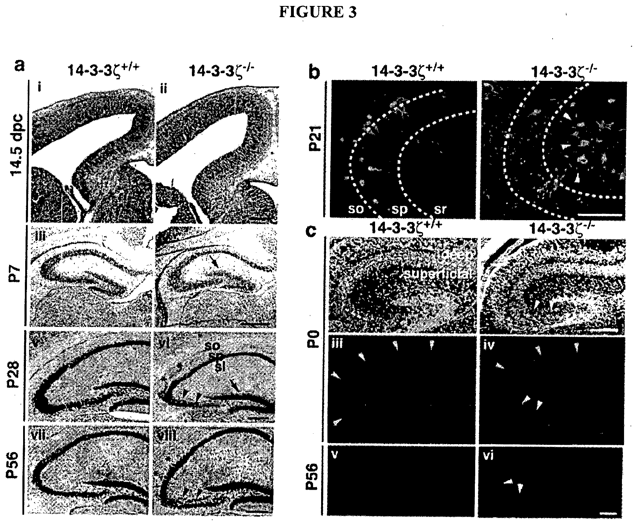

**Figure 3****: 14-3-3-ζ-deficient mice displayed lamination defects of the hippocampus.**
   Nissl staining shows the hippocampal development of WT and 14-3-3ζ^{062-/-} mice from 14.5 dpc until postnatal-day-56 (P56). Hippocampal cells are dispersed in the stratum pyramidale (sp) of the 14-3-3ζ^{062-/-} mice (iv, vi, viii). Arrowheads highlight the duplicated layer of the hippocampal pyramidal neurons in stratum radiatum (sr). Asterisks highlight the ectopically positioned pyramidal cells in the stratum oriens (so). Arrows indicate the loosely arranged granule neurons in the dentate gyrus. (b) Thyl-YFP transgene expression introduced in to the 14-3-3ζ⁰⁶² background revealed severe disorganization of hippocampal pyramidal neurons in 14-3-3ζ^{062-/-} mice. Blue, DAPI; green, Thyl expression. (c) Coronal sections of the hippocampus obtained from P0 (i-iv) and P56 (v-vi) mice of the indicated genotype. The deeper stratum pyramidale is populated by NeuN-positive pyramidal cells in WT hippocampi (iii, yellow arrowheads) forming a uniform mature zone from CA1 to CA3. In 14-3-3ζ^{062-/-} hippocampi, the maturation zone was less uniform with some NeuN-positive mature pyramidal cells ectopically positioned in both the deeper zone (yellow arrowheads) and superficial zone (white arrowheads) of the stratum pyramidale in CA3. In P56 14-3-3ζ^{062-/-} mice, immunostaining for NeuN highlighted pyramidal cells in the duplicated CA3 subfields indicating that ectopic cells achieved maturation (vi). Scale bars: 100um.

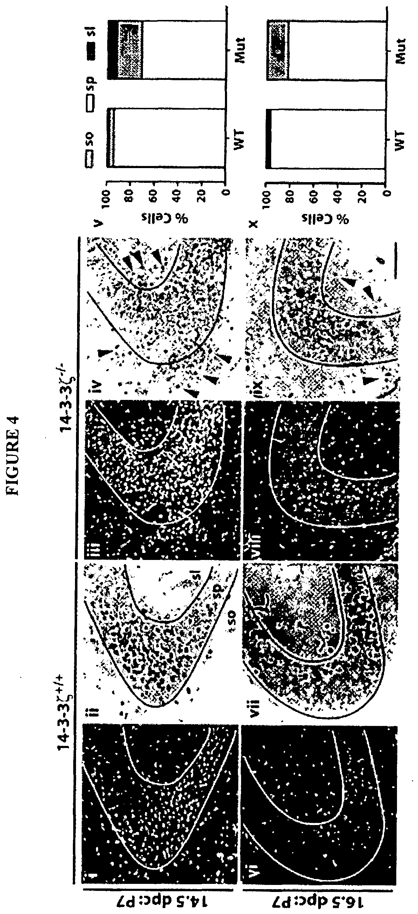

**Figure 4****: BrdU-pulse-chase analysis indicates neuronal migration defect in 14-3-3ζ-deficient mice.**
   BrdU-pulse-chase analysis at 14.5dpc:P7 (i-v) and 16.5dpc:P7 (vi-x) demonstrates that the BrdU-positive cells (black) locate within the stratum pyramidale (sp) in the CA3 subfield of WT hippocampi (ii & vii ). (v) Graph summarizes the percentage of the ectopic hippocampal neurons at 14.5dpc:P7. BrdU-labelled cells of 14-3-3ζ^{062-/-} mice were ectopically positioned. Neurons were stalled in the stratum oriens (so), or migrated beyond the stratum pyramidale and into the stratum lucidum (sl) (arrowheads in iv & ix). (x) Graph summarizes the percentage of the ectopic hippocampal neurons at 16.5dpc:P7. Scale bars: 100 µm
**Figure 5****: Abnormal mossy fibre pathways in 14-3-3ζ-deficient mice.**
   Calbindin immunostaining of the infrapyramidal (IPMF, yellow arrowheads) and the suprapyramidal (SPMF, white arrowheads) mossy fibre trajectories in 14-3-3ζ^{062+/+} (i,iii,v and vii) and 14-3-3ζ^{062-/-} (ii, iv, vi and viii) mice. Similar to WT controls, 14-3-3ζ^{062-/-} deficient neurites initially bifurcate into the SPMF and IPMF branches after navigating away from the dentate gyrus (DG). However, the IPMF branch of 14-3-3ζ^{062-/-} mice navigated aberrantly among the pyramidal cell somata (sp, white arrows). In addition, the diffuse SPMF branch of 14-3-3ζ^{062-/-} mice invaded the duplicated pyramidal cell layer in CA3. Scale bars = 100 µm.

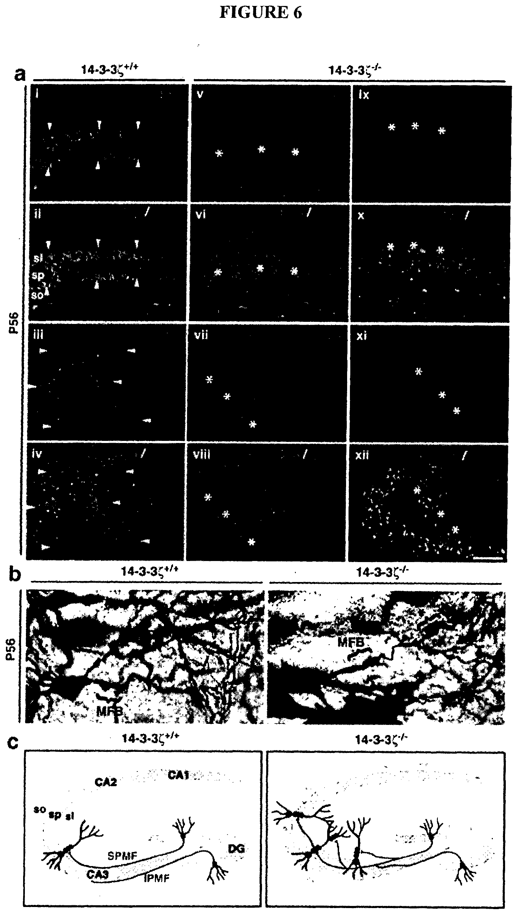

**Figure 6****: Functional synaptic connection between ectopic CA3 pyramidal cells and misrouted mossy fibres.**
   (i-iv) Hippocampal sections from P56 14-3-3ζ^{062+/+} mice stained with antibodies to synaptophysin (Syp) show immunoreactivity in both the IPMF (white arrowheads) and SPMF (yellow arrowheads). Syp staining is located in both the stratum oriens (so) and stratum lucidum (sl). surrounding the pyramidal somata of CA3. (v-viii) Syp staining of hippocampal sections from 14-3-3ζ^{062-/-} mice reveals that the mossy fibres navigating abnormally within the stratum pyramidale of CA3 (asterisks, v, vii ) form functional synapses. (ix-xii) Ectopic mature CA3 pyramidal cells (stained by NeuN; depicted with asterisks) communicate with the synaptic protein (Syp, green) from the misrouted mossy fibres. Scale bars = 100 µm. (b) Golgi stain reveals the dendritic arborization of the pyramidal cells of WT or 14-3-3ζ^{062-/-} adult mice (P35). A set of thorny excrescences, indicating the contact points with the misrouted mossy fibre synaptic boutons (MFB, bevelled line), is located on the apical proximal dendrites of CA3 pyramidal cells in WT neurons. Two sets of thorny excrescences are located on the apical dendritic tree in 14-3-3ζ^{062-/-} mice, one at the proximal apical dendrites and the other in the distal dendritic branches (*). (c) Schematic diagram depicts the misrouted mossy fibre trajectories and aberrant synaptic points of mossy fibre boutons communicating to the ectopic CA3 pyramidal cells in 14-3-3ζ^{062-/-} mice as compared to WT hippocampi.

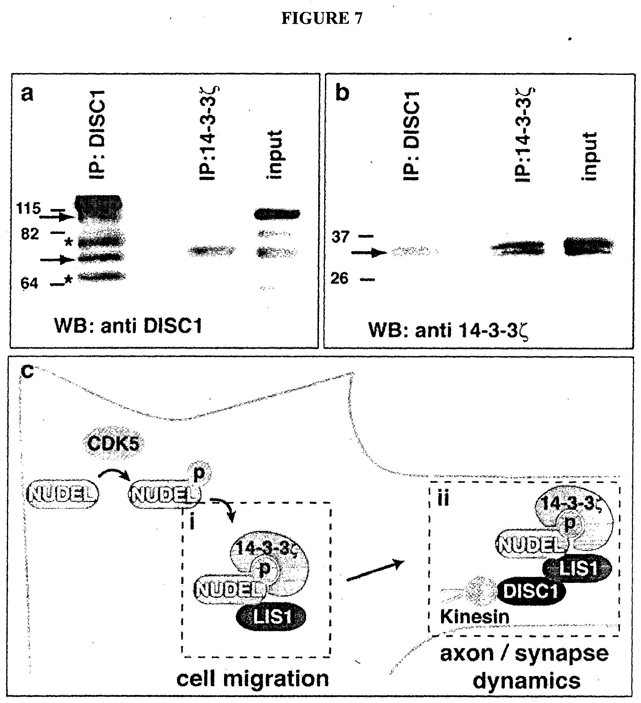

**Figure 7****: 14-3-3ζ^{062-/-} interacts with DISC1 to control neuronal development.**
   (a-b). Equal amounts of lysate from P7 mouse brains were immunoprecipitated with anti-DISC1 antibodies or anti-14-3-3 antibodies and immunoblotted with DISC1 (a), or EB1 purified antisera to recognize 14-3-3ζ (b). The relative expression levels of DISC1 isoforms and 14-3-3ζ from 5% of total cell lysate (input) used for co-immunoprecipitation were also determined by direct immunoblotting. Arrows indicate the major 100kDa and 75kDa bands of DISC1 (a) and 27kDa band representing 14-3-3ζ (b). Asterisk represents background IgG bands from immunoprecipitation. (c) Schematic representation of the role of 14-3-3ζ in neuronal migration and axonal growth. (i) 14-3-3ζ binds CDK5 phosphorylated Ndel1 to promote interaction with LIS1 and thereby promote neuronal migration. (ii) 14-3-3ζ is also present in the LIS1/Ndel1/DISC1 complex to control axonal growth dynamics.

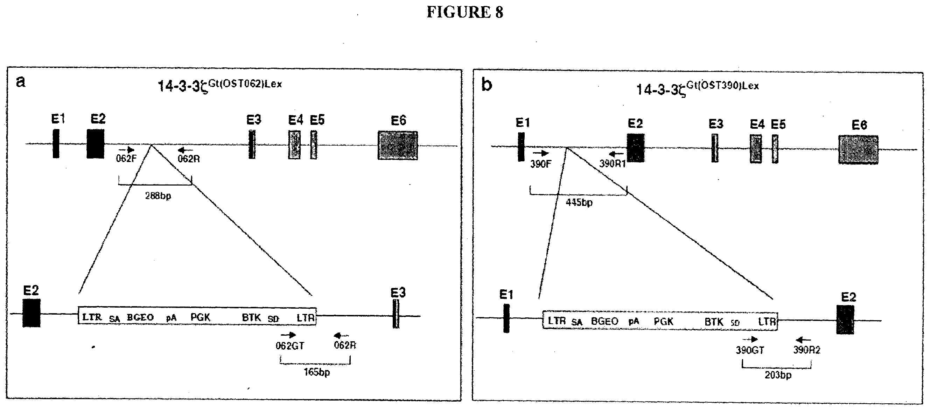

**Figure 8****: Gene trap mutation of the 14-3-3ζ gene.**
   Schematic showing the insertion point for mouse line 14-3-3ζ^{Gt(OST062)Lex}and (b) for mouse line 14-3-3ζ^{Gt(OST390)Lex}. The gene trap vector contains a splice acceptor sequence (SA) fused to a selectable marker gene (BGEO for 0 galactosidase/neomycin phosphotransferase fusion gene) that is thereby expressed under the endogenous 14-3-3ζ promoter. When integrated into the upstream exons of 14-3-3ζ BGEO produces a fusion transcript that interrupts mRNA transcription. The vectors also contain a PGK promoter followed by the first exon of Bruton's Tyrosine Kinase gene (BTK) upstream of a splice donor (SD) signal. BTK contains termination codons in all reading frames to prevent translation of downstream fusion transcripts. The gene trap vector is depicted in retrovirus form between two long terminal repeats (LTR). On both figures, arrows denote primers used for genotyping. Red boxes indicate non-coding untranslated sequence and green boxes denote coding sequence.

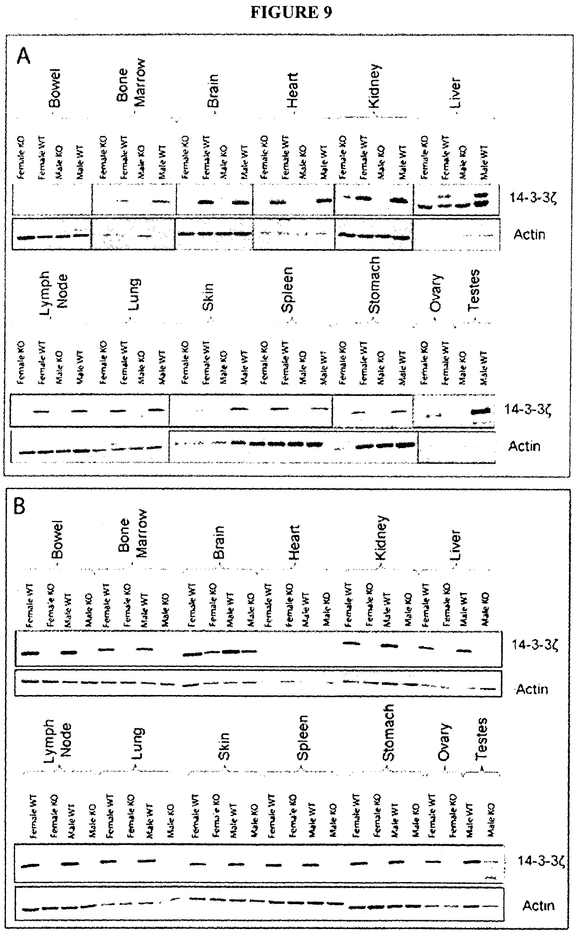

**Figure 9****: Western Blot analysis demonstrates that 14-3-3ζ expression is reduced in all tissues of mutant mice:**
   Tissues were harvested from (a) both male and female 14-3-3ζ^{062-/-} and age-matched 14-3-3ζ^{062+/+} mice and from (b) both male and female 14-3-3ζ^{390-/-} and age-matched 14-3-3ζ^{390+/+} mice. All samples were homogenised in NP40 lysis buffer containing protease inhibitors as described in the Materials and Methods. Protein concentrations were determined using Pierce BCA Protein Assay kit and 10µg protein was loaded per lane. Blots were probed with EB-1 antibody to detect 14-3-3ζ and anti- β-actin (1:5000) was used as a loading control. Bound antibodies were detected with HRP-conjugated secondary antibody (1:20,000, Pierce-Thermo Scientific). Immunoreactive proteins were visualized by ECL. Note that EB1 antibody may also detect 14-3-3 isoforms other than 14-3-3ζ.

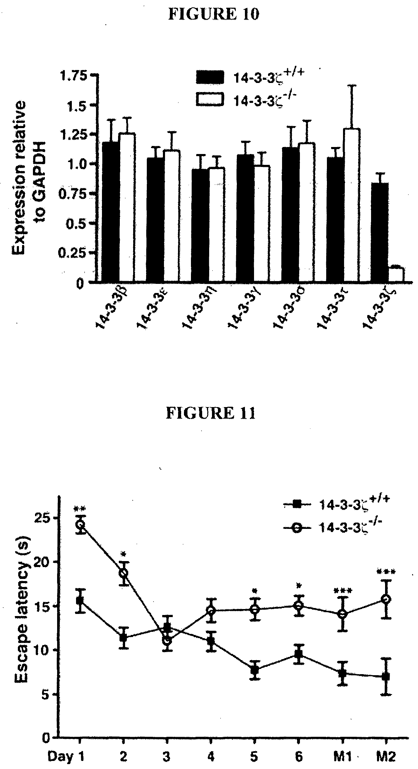

**Figure 10****: mRNA levels of 14-3-3 isoforms remain constant in 14-3-3ζ-deficient mouse brain:**
   Transcript levels of all 14-3-3 isoforms are unchanged in response to the deletion of the 14-3-3ζ isoform in brain tissue from 14-3-3ζ^{062-/-}mice. RNA was isolated from whole brain of three 14-3-3ζ^{062-/-}mice and three age-matched 14-3-3ζ^{062+/+} controls. Complementary DNA (cDNA) was generated from 1 µg RNA using Quantitect kit (Qiagen). Real Time PCR using Sybr Green (Qiagen) and Rotor Gene machines (Corbett) was used to determine levels of mRNA compared to GAPDH in samples for all isoforms of 14-3-3. See Table 1 for primer details.
**Figure 11****: 14-3-3ζ-deficient mice display cognitive dysfunction in learning and memory.**
   14-3-3ζ^{062-/-} mice (open circles; n=12) have lower capacity than 14-3-3ζ^{062+/+} mice (closed squares; n=12) for both spatial learning (Day 1-6) and memory in a cross maze escape task test. 14-3-3ζ^{062-/-} mice take longer to reach the escape platform throughout the training period and during the memory test phase (M1 and M2). Data from male and female mice is pooled. Error bars are presented as mean ± SEM. *, *p<* 0.05; ***, p* < 0.01; ****, p* < 0.001.
**Figure 12****: 14-3-3ζ-deficient mice display reduced startle reflex.** Startle amplitude of 14-3-3ζ^{062-/-} mice (open bar; n=13) is lower than 14-3-3ζ^{062+/+}mice (closed bars; n=14) over four pulse-alone blocks of 115 dB. The average (Avg) startle from all blocks is also shown.**, <0.05.
**Figure 13****: 14-3-3ζ expression is maintained in hippocampal neurons.**
   X-gal staining showing the endogenous expression of *14***-***3***-***3ζ* in P0 and P7 14-3-3ζ^{062+/-} hippocampus and cerebellum. The high level of 14-3-3ζ-lacZ expression in the hippocampus is evident in both the pyramidal neurons of the Ammon's horn and the mature dentate neurons but not in the cerebellum post-birth. Scale bar = 25 µm.

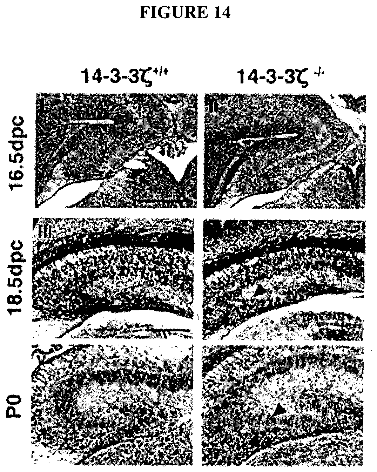

**Figure 14****. Hippocampal lamination defects in 14-3-34ζ-deficient mice.**
   Nissl staining shows the hippocampal development of WT (i, iii, v) and 14-3-3ζ^{062-/-}(ii, iv, vi) mice from 14.5 dpc until birth (P0). Hippocampal cells were dispersed in the stratum pyramidale (sp) of the 14-3- 3ζ^{062-/-} mice. Arrowheads highlight the duplicated layer of the hippocampal pyramidal neurons in stratum radiatum (sr). Asterisks highlight the ectopically positioned pyramidal cells in the stratum oriens (so). Scale bar = 25 µm.

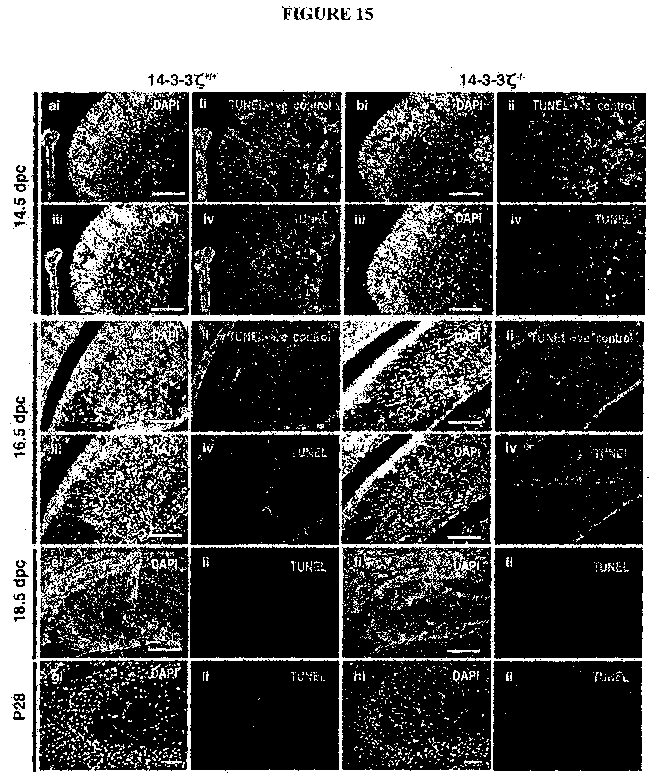

**Figure 15****: Mispositioned neurons in 14-3-3ζ-deficient mice survive into adulthood.** Apoptotic cells in hippocampal primordium (*a*-*f*) and mature hippocampi *(g-h).* No increase in fragmented, apoptotic cell nuclei (as shown in the green TUNEL positive cells in *aii* and *bii)* were detected 14-3-3ζ^{-/-} hippocampi. Scale bar = 100 µm.
**Figure 16****:**
   During development of the peripheral nervous system Nrp1-positive neural crest stem cells form the chromaffin (c), neurons (n) and glia (g) of the sympathathetic nervous system and adrenal glands, In contrast, Nrp2-positive neural crest cells form neurons and glia of the sensory nervous system. We have created transgenic mouse models expressing Cre and Red Fluorescent proteins from the Nrp1 promoter (Nrp1:Cre/RFP) or Cre and Green Fluorescent proteins from the Nrp2 promoter (Nrp2:Cre/GFP). These mice facilitate the spectral separation of Nrp1 and Nrp2 positive neural stem cells that can be used to purify each subpopulation.
**Figure 17****:**
   Coronal section of a P0 mouse brain from a Nrp2:Cre/GFP mouse stained for Beta galactosidase. (B) higher magnification of boxed area in (A) demonstrates that the cornu ammonis (CA1-3) pyramidal neurons and dentate gyrus (DG) granular neurons of the hippocampus (h) are derived from Nrp2 expressing neural stem cells. Nrp2 is also expressed in neural stem cells in the ventricular zone (VZ).

### DETAILED DESCRIPTION OF THE INVENTION

The present invention is predicated, in part, on the determination that a reduction in the functional level of protein 14-3-3ζ, such as in the context of absolute levels of protein 14-3-3ζ or levels of protein 14-3-3ζ/DISC1 complex formation, is indicative of the onset or predisposition to the onset of a neuropsychiatric condition, such as schizophrenia or related condition. However, the further determination that this leads to the degeneration of the hippocampus has provided the basis for developing a therapeutic treatment for individuals exhibiting a defective hippocampus, such as schizophrenia patients. The still further determination that a subpopulation of neural crest stem cells selectively differentiates to neurons of the hippocampus and can be grafted into the brain to effect regeneration of the hippocampus has now led, by virtue of the combination of all these findings, to the development of a treatment regime for conditions such as schizophrenia.

Accordingly, one aspect of the present disclosure is directed to a method of treating a mammal with a condition characterised by a defective hippocampus, said method comprising administering to said mammal an effective number of Nrp2⁺ neural crest stem cells or mutants or variants thereof for a time and under conditions sufficient to effect regeneration of the hippocampus.

Reference to "hippocampus" should be understood as a reference to the hippocampus region of the brain. Without limiting the present invention to any one theory or mode of action the hippocampus is a major component of the brains of humans and other mammals. It belongs to the limbic system and plays important roles in the consolidation of information from short-term memory to long-term memory and spatial navigation. Like the cerebral cortex, with which it is closely associated, it is a paired structure, with mirror-image halves in the left and right sides of the brain. In humans and other primates, the hippocampus is located inside the medial temporal lobe, beneath the cortical surface. It contains two main interlocking parts: Ammon's horn and the dentate gyrus.

Anatomically, the hippocampus is an elaboration of the edge of the cerebral cortex (Amaral and Lavenex (2006). "Ch 3. Hippocampal Neuroanatomy". The Hippocampus Book. Oxford University Press. The structures that line the edge of the cortex make up the so-called limbic system (Latin *limbus* = *burder*): these include the hippocampus, cingulate cortex, olfactory cortex, and amygdala. The hippocampus is anatomically connected to parts of the brain that are involved with emotional behaviour-the septum, the hypothalamic mammillary body, and the anterior nuclear complex in the thalamus.

The hippocampus as a whole has the shape of a curved tube, which has been analogized variously to a seahorse, a ram's horn (*Cornu Ammonis,* hence the subdivisions CA I through CA4), or a banana (Amaral and Lavenex, *supra).* It can be distinguished as a zone where the cortex narrows into a single layer of densely packed pyramidal neurons which curl into a tight U shape; one edge of the "U," field CA4, is embedded into a backward facing strongly flexed V-shaped cortex, the dentate gyrus. It consists of ventral and dorsal portions, both of which share similar composition but are parts of different neural circuits (Moser and Moser (1998) Hippocampus 8(6): 608-19). This general layout holds across the full range of mammalian species.

The entorhinal cortex (EC), located in the parahippocampal gyrus, is considered to be part of the hippocampal region because of its anatomical connections. The EC is strongly and reciprocally connected with many other parts of the cerebral cortex. In addition, the medial septal nucleus, the anterior nuclear complex and nucleus reuniens of the thalamus and the supramammillary nucleus of the hypothalamus, as well as the raphe nuclei and locus coeruleus in the brainstem send axons to the EC. The main output pathway (perforant path) of EC axons comes from the large stellate pyramidal cells in layer II that "perforate" the subiculum and project densely to the granule cells in the dentate gyrus, apical dendrites of CA3 get a less dense projection, and the apical dendrites of CA1 get a sparse projection. Thus, the perforant path establishes the EC as the main "interface" between the hippocampus and other parts of the cerebral cortex. The dentate granule cell axons (called mossy fibers) pass on the information from the EC on thorny spines that exit from the proximal apical dendrite of CA3 pyramidal cells. Then, CA3 axons exit from the deep part of the cell body, and loop up into the region where the apical dendrites are located, then extend back into the deep layers of the entorhinal cortex-the Shaffer collaterals completing the reciprocal circuit; field CA1 also sends axons back to the EC, but these are more sparse than the CA3 projection. Within the hippocampus, the flow of information from the EC is largely unidirectional, with signals propagating through a series of tightly packed cell layers, first to the dentate gyrus, then to the CA3 layer, then to the CA1 layer, then to the subiculum, then out of the hippocampus to the EC, mainly due to collateralization of the CA3 axons. Each of these layers also contains complex intrinsic circuitry and extensive longitudinal connections (Amaral and Lavenex 2006, *supra).*

Several other connections play important roles in hippocampal function (Amaral and Lavenex 2006, *.supra*)*.* Beyond the output to the EC, additional output pathways go to other cortical areas including the prefrontal cortex. A very important large output goes to the lateral septal area and to the mammillary body of the hypothalamus. The hippocampus receives modulatory input from the serotonin, norepinephrine, and dopamine systems, and from nucleus reuniens of the thalamus to field CA1. A very important projection comes from the medial septal area, which sends cholinergic and GABAergic fibers to all parts of the hippocampus. The inputs from the septal area play a key role in controlling the physiological state of the hippocampus: destruction of the septal area abolishes the hippocampal theta rhythm, and severely impairs certain types of memory(Winson (1978), Science 201(4351):160-63).

The cortical region adjacent to the hippocampus is known collectively as the parahippocampal gyrus (or parahippocampus) (Eichenbaum et al. (2007), Annu Rev Neurosci 30:123-52). It includes the EC and also the perirhinal cortex, which derives its name from the fact that it lies next to the rhinal sulcus. The perirhinal cortex plays an important role in visual recognition of complex objects, but there is also substantial evidence that it makes a contribution to memory which can be distinguished from the contribution of the hippocampus, and that complete amnesia occurs only when both the hippocampus and the parahippocampus are damaged (Eichenbaum et al. (2007), Annu Rev Neurosci 30:123-52).

Reference to a "defective" hippocampus should be understood as a reference to a hippocampus, all or part of the structure or function which is not normal. To this end, the defect may be congenital or it may be acquired. For example, anatomical malformation of the hippocampus may be present from birth. However, the hippocampus defects which are associated with the onset of many neuropsychiatric and neurodegenerative conditions are often acquired postnatally and are the result of injuries (e.g. head trauma or asphyxiation), exposure to environmental factors, drug use and the like. In other situations, a genetic defect is present congenitally but does not manifest until much later sometimes not until adulthood. As detailed hereinbefore, the method of the present disclosure provides a means of regenerating hippocampus tissue, thereby at least in part restoring tissue which is structurally and functionally normal. In this context, reference to "regeneration" is a reference to the generation of at least some normal hippocampus tissue within the hippocampus area of the brain. It is not intended to mean that the hippocampus is entirely replaced or that even all of the defective tissue is replaced. Rather, it is a reference to the fact that the method of the present disclosure increases the proportion of normal hippocampus tissue relative to the proportion which existed in the subject prior to the application of the method of the disclosure. Accordingly, the method of the present disclosure isnot limited to its application in the context of the complete normalisation of all the affected hippocampus tissue. Rather, it should also be understood to extend to the partial normalisation of all or only some of the defective tissue

The term "mammal" as used herein includes humans, primates, livestock animals (e.g. horses, cattle, sheep, pigs, donkeys), laboratory test animals (e.g. mice, rats, guinea pigs), companion animals (e.g. dogs, cats) and captive wild animals (e.g. kangaroos, deer, foxes). Preferably, the mammal is a human or a laboratory test animal. Even more preferably, the mammal is a human.

According to this embodiment there is provided a method of treating a human with a condition characterised by a defective hippocampus, said method comprising administering to said mammal an effective number of Nrp2⁺ neural crest stem cells or mutants or variants thereof for a time and under conditions sufficient to effect regeneration of the hippocampus.

As detailed above, the method of the present disclosure is predicated on the determination that the administration of Nrp2⁺ neural crest stem cells to the brain of a mammal with a defective hippocampus results in not just engraftment of the cells into the tissue, but also repair and restoration of hippocampus morphology and functioning. By "stem cell" is meant that the cell is not fully differentiated but requires further differentiation to achieve maturation. Such cells are also sometimes referred to as "precursor" cells, "progenitor" cells, "multipotent" cells or "pluripotent" cells.

Without limiting the present invention to any one theory or mode of action. neural crest cells are a transient, multipotent, migratory cell population unique to vertebrates that give rise to a diverse cell lineage including melanocytes, craniofacial cartilage and bone, smooth muscle, peripheral and enteric neurons and glia. After gastrulation, neural crest cells are specified at the border of the neural plate and the non-neural ectoderm. During neuralation, the borders of the neural plate, also known as the neural folds, converge at the dorsal midline to form the neural tube. Subsequently, neural crest cells from the roof plate of the neural tube undergo an epithelial to mesenchymal transition, delaminating from the neuroepithelium and migrating through the periphery where they differentiate into varied cell types. Underlying the development of the neural crest is a gene regulatory network, described as a set of interacting signals, transcription factors, and downstream effector genes that confer cell characteristics such as multipotency and migratory capabilities.

Reference to "neural crest stem cell" should therefore be understood as a reference to any cell which exhibits one or more of the functional or phenotypic characteristics of a neural crest stem cell or which exhibits the potentiality to differentiate to any of the cell types which a neural crest stem cell can differentiate to. The subject neural crest stem cell may be one which exhibits multipotentiality, for example is a progenitor which can be induced to differentiate to give rise to any one or more multiple peripheral structures such as the cranial skeleton, dentine of the teeth, melanocytes, peripheral neurons, adrenal chromafin cells and specific cells within hair follicles, or it may be already committed to a subgroup of these lineages. However, despite this initial level of commitment, the subject cell is nevertheless still a "stem cell" on the basis that it is not fully differentiated. The use of the term "stem cell" should not be understood as a limitation on the maturity/immaturity of the subject cell relative to that which might be implied by the use of the terms "progenitor cell", "multipotent cell", "pluripotent cell" or other such term.

Reference to a cell exhibiting a "functional" characteristic of a neural crest stem cell should be understood as a reference to a cell which is restricted to differentiating along any one or more of the neural crest cell derived lineages, such as those detailed above. Reference to a "phenotypic" characteristic should be understood as a reference to a cell surface or intracellular expression profile of one or more proteinaceous or non-proteinaceous molecules which is characteristic of a neural crest stem cell. To this end, in accordance with the method of the present disclosure, it has now been determined that it is neural crest stem cells which express Nrp2 (neuropilin 2) which selectively give rise to functional neurons of the hippocampus and are therefore the source of cells for regeneration of the hippocampus. Reference to "Nrp2⁺" should therefore be understood as a reference to a neural crest stem cell which is characterised by cell surface expression of Nrp2.

Still without limiting the present invention in any way, neural crest stem cells can be derived either from an embryonic source or, more conveniently, from an adult source. Specifically, adult neural crest stem cells can be easily and routinely isolated from the dentine of teeth and the bulge of the hair follicle and provide the same precursor cell source for the neurons and glia in the central nervous system. When engrafted, these cells differentiate into GABAergic neurons and oligodendrocytes. Accordingly, either an adult source or an embryonic source can be used in the context of the method of the present disclosure. In one embodiment, the subject stem cells are adult stem cells.

According to this embodiment, there is therefore provided a method of treating a mammal with a condition characterised by a defective hippocampus, said method comprising administering to said mammal an effective number of adult Nrp2⁺ neural crest stem cells or mutants or variants thereof for a time and under conditions sufficient to effect regeneration of the hippocampus.

In yet another embodiment, said adult Nrp2⁺ neural crest stem cells are isolated from the dentine or the hair follicle.

The subject Nrp2⁺ neural crest stem cells population may be a single cell suspension or a cell aggregate, such as a tissue, which has been freshly isolated from an individual (such as an individual who may be the subject of treatment) or it may have been sourced from a non-fresh source, such as from a culture (for example, where cell numbers were expanded and/or the cells were cultured so as to render them receptive to differentiative signals) or a frozen stock of cells (for example, an established cell line), which had been isolated at some earlier time point either from an individual or from another source. It should also be understood that the subject cells may have undergone some other form of treatment or manipulation, such as but not limited to enrichment or purification, modification of cell cycle status, molecular transformation or the formation of a cell line. Accordingly, the subject cell may be a primary cell or a secondary cell. A primary cell is one which has been freshly isolated from an individual. A secondary cell is one which, following its isolation, has undergone some form of *in vitro* manipulation such as the preparation of a cell line.

Reference to a "mutant or variant" of the subject cellular population should be understood as a reference to a cell which is derived from the cellular population but exhibits at least one difference at the phenotypic or functional level. For example, the mutant or variant may have altered expression of its cell surface markers as a whole or some aspect of its functionality subsequently to initial isolation. Such changes can occur either spontaneously (as exemplified by the spontaneous upregulation or downregulation of cell surface markers which can occur subsequently to *in vitro* culture or spontaneous transformation) or as a result of a directed manipulation, such as would occur if a cell was deliberately transformed (for example, in order to effect the creation of a cell line) or transfected (for example to effect the expression of a particular gene or marker).

It should be understood that the Nrp2⁺ neural crest stem cell populations of the present invention may exhibit some variation in differentiative status within a single phenotypic profile. That is, within a single phenotypic profile, although the cells comprising that profile may substantially exhibit similar phenotypic and/or functional characteristics, there may nevertheless exhibit some differences. This may be apparent, for example, in terms of differences in the transcriptome profile or cell surface marker expression (other than the markers defined herein) of the cells which comprise the phenotypic profile in issue. For example, the Nrp2⁺ neural crest stem cells may not represent a highly specific and discrete stage, but may be characterised by a number of discrete cellular subpopulations which reflect a transition or phase if one were to compare cells which have differentiated into this stage versus cells which are on the cusp of maturing out of this stage. Accordingly, the existence of cellular subpopulations within a single phenotypic profile of the present invention is encompassed.

To the extent that human embryonic stem cells are sought to be isolated and differentiated, *in vitro,* to a Nrp2⁺ neural crest stem cell, these cells may be derived from the inner cell mass of a blastocyst stage human embryo or an established cell line may be used (such as those developed by Thomson and Odorico, Trends Biotechnol., 18:53-57 (2002). namely. H1. H7, H9.1, H9.2, H13 or H14). To generate human embryonic stem cell cultures *de novo,* cells from the inner cell mass are separated from the surrounding trophectoderm by microsurgery or by immunosurgery (which employs antibodies directed to the trophectoderm to break it down) and are plated in culture dishes containing growth medium supplemented with fetal bovine serum (alternatively. KnockOut Dulbecco's modified minimal essential medium containing basic FGF can be supplemented with Serum Replacer (Life Technologies) and used without serum), usually on feeder layers of mouse embryonic fibroblasts that have been mitotically inactivated to prevent replication. Alternatively, a feeder-free culture system may be employed, such as that reported by Chunhui Xu, Melissa Carpenter and colleagues using Matrigel or laminin as a substrate, basic FGF, and conditioned medium from cultures of mouse embryo fibroblasts (Xu, et al., Nat Biotechnol. 2001 Oct; 19(10):971-4). The Nrp2⁺ neural crest stem cell population is then differentiated from this starting pluripotent stem cell population. Insofar as the mentioned human embryonic stem cells (hESC) are obtained by destruction of a human embryo, either directly or indirectly via a cell line that involved the destruction of a human embryo, the hESC do not form part of the claimed invention and are mentioned for reference purposes only.

The present disclosure is predicated on administering a Nrp2⁺ neural crest stem cell population to a mammal in order to facilitate its localisation to the brain of the mammal. By "localisation" is meant that at least some of the Nrp2⁺ neural crest stem cell population which is introduced to the patient targets the brain. It should be understood, however, that in terms of any treatment event, a proportion of the administered Nrp2⁺ neural crest stem cells may not target the brain, but may either be cleared or else lodge in non-brain tissues.

The cells which are administered in the context of the present disclosure are preferably autologous cells which are isolated and transplanted back into the individual from which they were originally harvested (for example, dentine derived Nrp2⁺ neural crest stem cells), However, it should be understood that the present disclosure nevertheless extends to the use of cells derived from any other suitable source where the subject cells exhibit the same major histocompatability profile as the individual who is the subject of treatment. Accordingly, such cells are effectively autologous in that they would not result in the histocompatability problems which are normally associated with the transplanting of cells exhibiting a foreign MHC profile. Such cells should be understood as falling within the definition of "autologous". For example, under certain circumstances it may be desirable, necessary or of practical significance that the subject cells are isolated from a genetically identical twin, or are differentiated from the stem cells of an embryo generated using gametes derived from the subject individual or cloned from the subject individual. The cells may also have been engineered to exhibit the desired major histocompatability profile. The use of such cells overcomes the difficulties which are inherently encountered in the context of tissue and organ transplants.

However, where it is not possible or feasible to isolate or generate autologous cells, it may be necessary to utilise allogeneic cells. "Allogeneic" cells are those which are isolated from the same species as the subject being treated but which exhibit a different MHC profile. Although the use of such cells in the context of therapeutics may result in the onset of an allogeneic based immune response, this problem can nevertheless be minimised by use of cells which exhibit an MHC profile exhibiting similarity to that of the subject being treated, such as a cell population which has been isolated/generated from a relative such as a sibling, parent or child. The immunological tissue rejection which is often characteristic of the use of allogeneic cells may also be minimised via the use of immunosuppressant drugs. However, whether or not the use of such drugs is deemed necessary will depend on the particular circumstances of each case. Also contemplated herein is the use of established Nrp2⁺ neural stem cell lines. The present disclosure should also be understood to extend to xenogeneic transplantation. That is, the cells which are introduced into a patient are isolated from a species other than the species of the subject being treated.

Reference to an "effective number" means that number of cells necessary to at least partly attain the desired effect, or to delay the onset of, inhibit the progression of, or halt altogether the onset or progression of the particular condition being treated. Such amounts will depend, of course, on the particular condition being treated, the severity of the condition and individual patient parameters including age, physical conditions, size, weight, physiological status, concurrent treatment, medical history and parameters related to the disorder in issue. One skilled in the art would be able to determine the number of Nrp2⁺ neural crest stem cells that would constitute an effective dose, and the optimal mode of administration thereof without undue experimentation, this latter issue being further discussed hereinafter. These factors are well known to those of ordinary skill in the art and can be addressed with no more than routine experimentation. It is preferred generally that a maximal cell number be used, that is, the highest safe number according to sound medical judgement. It will be understood by those of ordinary skill in the art, however, that a lower cell number may be administered for medical reasons, psychological reasons or for any.other reasons.

It should also be understood that not all of the Nrp2⁺ neural crest stem cells which are administered in accordance with the method of the disclosure may necessarily contribute to the treatment regime discussed herein. For example, some cells may localise to non brain tissues while others may become non-viable or non-functional. In another example, where the Nrp2⁺ neural crest stem cell population has been purified from a heterogeneous cellular population (such as a hair follicle sample), the purified population may nevertheless comprise some non-Nrp2⁺ neural crest stem cells where 100% purity is not obtained, The present disclosure is therefore achieved provided the relevant portion of the cells which are introduced to the patient constitute an "effective number" as defined above.

In the context of this aspect of the present disclosure, the subject cells require introduction into the subject individual. To this end, the cells may be introduced by any suitable method. For example, cell suspensions may be introduced by direct injection to a tissue or inside a blood clot whereby the cells are immobilised in the clot thereby facilitating transplantation. The cells may also be encapsulated prior to transplantation. Encapsulation is a technique which is useful for preventing the dissemination of cells which may continue to proliferate (i.e. exhibit characteristics of immortality). The cells may also be introduced by localised, intravenous or systemic routes.

The cells may also be introduced by surgical implantation (grafting). This may be necessary, for example, where the cells exist in the form of a tissue graft or where the cells are encapsulated prior to transplanting. Without limiting the present disclosure to any one theory or mode of action, where cells are administered as an encapsulated cell suspension, the cells will coalesce into a mass.

The cells which are administered to the patient can be administered as single or multiple doses by any suitable route. Preferably, and where possible, a single administration is utilised, particularly where surgical engraftment into the brain is the method used. Administration via injection can be directed to various regions of a tissue or organ, depending on the type of treatment required.

In accordance with the method of the present disclosure, other proteinaceous or non-proteinaceous molecules such as antibiotics or differentiation inducing cytokines may be coadministered either with the introduction of the Nrp2⁺ neural crest stem cells or during the differentiation and proliferation phase of the transplanted cells. By "coadministered" is meant simultaneous administration in the same formulation or in different formulations via the same or different routes or sequential administration via the same or different routes. By "sequential" administration is meant a time difference of from seconds, minutes, hours or days between the transplantation of these cells and the administration of the proteinaceous or non-proteinaceous molecules. For example, it may be desirable to co-administer molecules which will facilitate the localisation or the directed differentiation of the subject Nrp2⁺ neural crest stem cells. Other examples of circumstances in which co-administration may be required include, but are not limited to:

When administering non-syngeneic cells or tissues to a subject, there usually occurs immune rejection of such cells or tissues by the subject. In this situation it would be necessary to also treat the patient with an immunosuppressive regimen, preferably commencing prior to such administration, so as to minimise such rejection. Immunosuppressive protocols for inhibiting allogeneic graft rejection, for example via administration of cyclosporin A, immunosuppressive antibodies, and the like are widespread and standard practice.

Depending on the nature of the condition being treated, it may be necessary to maintain the patient on a course of medication to alleviate the symptoms of the condition until such time as the transplanted cells become integrated and fully functional (for example, the administration of anti-psychotic drugs to treat schizophrenia). Alternatively, at the time that the condition is treated, it may be necessary to commence the long term use of medication to prevent re-occurrence of the damage. For example, where the subject damage was caused by an autoimmune condition, the ongoing use of immunosuppressive drugs may be required even when syngeneic cells have been used.

It should also be understood that the method of the present disclosure can either be performed in isolation to treat the condition in issue or it can be performed together with one or more additional techniques designed to facilitate or augment the subject treatment. These additional techniques may take the form of the co-administration of other proteinaceous or non-proteinaceous molecules, as detailed hereinbefore.

Reference to a "condition characterised by a defective hippocampus" should be understood as a reference to any condition, a symptom or cause of which is hippocampus degeneration or damage. Examples, of such conditions include, but are not limited to, congenital anatomical abnormalities of the brain, acquired injury such as through head trauma or asphyxiation, atrophy and hypoplasia such as that seen in returning military officers after extended duress or conditions characterised by a reduction in the level of functional protein 14-3-3ζ or protein 14-3-3ζ/DISC1 complex formation, such as a neuropsychiatric condition.

Reference to a "neuropsychiatric condition" should be understood as a reference to a condition characterised by neurologically based cognitive, emotional and behavioural disturbances. Examples of such conditions include, *inter alia,* a condition characterised by one or more symptoms of schizophrenia, schizophrenia, schizotypal personality disorder, psychosis, bipolar disorder, manic depression, affective disorder, or schizophreniform or schizoaffective disorders, psychotic depression autism, drug induced psychosis, delirium, alcohol withdrawal syndrome or dementia induced psychosis.

In one embodiment, said neuropsychiatric condition is a condition which is characterised by one or more symptoms of schizophrenia.

According to this embodiment, there is provided a method of treating a mammal with a neuropsychiatric condition, said method comprising administering to said mammal an effective number of Nrp2⁺ neural crest stem cells or mutants or variants thereof for a time and under conditions sufficient to effect regeneration of the hippocampus.

In one embodiment, said mammal is a human. In another embodiment, said Nrp2⁺ neural crest stem cells are adult-derived stem cells.

In still another embodiment, said neuropsychiatric condition is a condition characterised by one or more symptoms of schizophrenia.

In a further embodiment, said condition is schizophrenia.

Reference to "symptoms characteristic of schizophrenia" should be understood as a reference to any one or more symptoms which may occur in an individual suffering from schizophrenia. These symptoms may be evident throughout the disease course or they may be evident only transiently or periodically. For example, the hallucinations associated with schizophrenia usually occur in periodic episodes while the characteristic social withdrawal may exhibit an ongoing manifestation. It should also be understood that the subject symptoms may not necessarily be exhibited by all individuals suffering from schizophrenia. For example, some individuals may suffer from auditory hallucinations only while others may suffer only from visual hallucinations. However, for the purpose of the present invention, any such symptoms, irrespective of how many or few schizophrenia patients ever actually exhibit the given symptom, are encompassed by this definition. Without limiting the present invention to any one theory or mode of action, the symptoms that are most commonly associated with the disease are called positive symptoms (which denote the presence of grossly abnormal behaviour), thought disorder and negative symptoms. Thought disorder and positive symptoms include speech which is difficult to follow or jumping from one subject to another with no logical connection, delusions (false beliefs of persecution, guilt, grandeur or being under outside control) and hallucinations (visual or auditory). Thought disorder is the diminished ability to think clearly and logically. Often it is manifested by disconnected and nonsensical language that renders the person with schizophrenia incapable of participating in conversation, contributing to alienation from family, friends and society. Delusions are common among individuals with schizophrenia. An affected person may believe that he or she is being conspired against (called "paranoid delusion"). "Broadcasting" describes a type of delusion in which the individual with this illness believes that their thoughts can be heard by others. Hallucinations can be heard, seen or even felt. Most often they take the form of voices heard only by the afflicted person. Such voices may describe the person's actions, warn of danger or tell him what to do. At times the individual may hear several voices carrying on a conversation. Less obvious than the "positive symptoms" but equally serious are the deficit or negative symptoms that represent the absence of normal behaviour. These include flat or blunted affect (i.e. lack of emotional expression); apathy, social withdrawal and lack of insight. Both the positive symptoms and the negative symptoms should be understood to fall within the definition of "symptoms characteristic of schizophrenia".

In addition to the fact that there may be significant variation between schizophrenia patients in terms of which symptoms they exhibit, it should also be understood that there are other neuropsychiatric conditions which are also characterised by one or more of these symptoms. Hallucinations, for example, are also commonly observed in patients with bipolar disorder, psychotic depression, delirium and dementia induced psychosis, for example. Accordingly, reference to a condition characterised by one or more symptoms characteristic of schizophrenia should be understood as a reference to any neuropsychiatric condition which is characterised by the presence of one or more of these symptoms. In one embodiment, said condition is schizophrenia.

In a related aspect of the present invention, the subject undergoing treatment may be undergoing therapeutic or prophylactic treatment and may be any human or animal in need of therapeutic or prophylactic treatment. In this regard, reference herein to "treatment" and "prophylaxis" is to be considered in its broadest context. The term "treatment" does not necessarily imply that a mammal is treated until total recovery. Similarly, "prophylaxis" does not necessarily mean that the subject will not eventually contract a disease condition. Accordingly, treatment and prophylaxis include amelioration of the symptoms of a particular condition or preventing or otherwise reducing the risk of developing a particular condition. The term "prophylaxis" may be considered as reducing the severity of the onset of a particular condition. "Treatment" may also reduce the severity of an existing condition.

Yet another aspect of the present disclosure is directedto the use of Nrp2⁺ neural crest stem cells or mutants or variants thereof in the manufacture of a medicament for the treatment of a condition in a mammal, which condition is characterised by a defective hippocampus, wherein said stem cells regenerate the hippocampus.

In one embodiment said mammal is a human.

In another embodiment, said Nrp2⁺ neural crest stem cells are adult stem cells and still more particularly dentine or hair follicle derived stem cells.

In a further embodiment, said condition is a congenital anatomical abnormality of the brain, acquired brain injury such as through head trauma or asphyxiation or a condition characterised by a reduction in the level of functional protein 14-3-3ζ or protein 14-3-3ζ/DISC1 complex formation.

In anther embodiment, said condition is a neuropsychiatric condition, more particularly a condition characterised by one or more symptoms of schizophrenia, schizophrenia, schizotypal personality disorder, psychosis, bipolar disorder, manic depression. affective disorder, or schizophreniform or schizoaffective disorders, psychotic depression, autism, drug induced psychosis, delirium, alcohol withdrawal syndrome or dementia induced psychosis.

Yet another aspect of the present disclosure is directed to an isolated cellular population comprising Nrp2⁺ neural crest stem cells for use in the method of the disclosure.

The present invention is further described by reference to the following non-limiting examples.

### EXAMPLE 1

### Materials and Methods

**Mice.** 14-3-3ζ^{Gt(OST062)Lex} and 14-3-3ζ^{Gt(OST390)Lex} mutant mice carrying gene trap constructs that contain the Geo reporter gene were derived from Lexicon Genetics ES cell lines OST062 and OST390, respectively. The gene trap vector in 14-3-3ζ^{Gt(OST062)Lex} mice inserted into the first intron of *14-3-3ζ*, whereas the gene trap vector in 14-3-3ζ^{Gt(OST390)Lex} mice inserted into the second intron of *14-3-3ζ.* ES cell lines were amplified and injected into SV129 blastocysts. Resulting germ line transmitting males were either maintained in the SV129 background or backcrossed in to the C57/B16 and BA1, BC backgrounds over 6 generations. qRT-PCR and western blot from whole tissue samples was used to confirm complete KO of the gene in these mouse strains. *14-3-3C* genotype was determined by PCR amplification of genomic tail DNA using the primers detailed in supplementary table 1. The WT allele amplified a band of 288 bp (14-3-3ζ^{Gt(OSTO62lex}) or 445 bp (14-3-3ζ^{Gt(OST390)Lex})and the mutant gene trapped allele amplified a band of 165 bp (14-3-3ζ^{(Gt(OST062)Lex}) or 203 bp (14-3-3ζ^{Gt(OST390)Lex}). Mice were maintained as heterozygous breeding pairs that were phenotypically indistinguishable to WT littermates. Animal experiments were conducted in accordance with the guidelines of the Animal Ethics Committee of the Institute of Medical and Veterinary Sciences and the University of Adelaide.

**Behavioural assays.** All procedures were carried out under normal light conditions between 8.00 am and 12.00 pm. Behavioural phenotyping was performed as previously described (Coyle et al. Behav Brain Res 2009, 197(1): 210-218; Summers et al. Pediatr Res 2006; 59(1): 66-71; van den Buuse et al. Int J Neuropsychopharmacol 2009; 12(10): 1383-1393). One cohort of mice was used for the open field test at ages of 5-, 10-, 20- and 40-week time points. One cohort of mice was used at the age of 12 weeks for spatial working memory, then elevated plus maze and object recognition tasks. A separate cohort of mice was used at the age of 12 weeks for PPI.

**Locomotor function test.** Exploratory activity and anxiety level of mice were measured in an open field made from a box (50 cm x 27 cm) with the floor divided into 15 squares (9 cm x 10 cm). Each mouse was introduced in to the same position of the box facing the right top corner. The behaviour of the mouse was observed for 3 min and locomotor activity was scored as a measure of line crossings (i.e. when a mouse removed all four paws from one square into another). Number of rears up was scored when a mouse had both front paws off the floor. Urine and faecal material were removed between session and the box was cleaned thoroughly with 80% ethanol to remove any lingering scents.

**Object recognition test**. The object recognition task takes advantage of the natural affinity of mice for novelty; mice that recognise a previously seen (familiar) object will spend more time exploring novel objects (Dere et al. Neurosci Biobehav Rev 2006; 30(8):1206-1224; Sik et al. Behav Brain Res 2003; 147(1-2):49-54). Briefly, the apparatus consisted of a plastic arena (length; 50 cm, width; 35 cm, depth; 20 cm) filled with bedding. Two different sets of objects were used; a yellow -capped plastic jar (height, 6 cm; base diameter, 4.3 cm) and a red plastic bulb (length: 8 cm, width: 4 cm). Mice spent equal amounts of time when presented with both of these objects, regardless of the position they were placed in the arena (data not shown). At 12 weeks of age the same cohort of mice tested for spatial learning and memory were assessed for object recognition memory. Each mouse was given 5-min to explore the test box without any objects present to habituate them to the test arena. Mice underwent the testing session comprised of two trials. The duration of each trial was 3 min. During the first trial (the sample phase), the box contained two identical objects (a, samples) which were placed in the north-west (left) and northeast (right) corners of the box (5 cm away from the walls). A mouse was always placed in the apparatus facing the south wall. After the first exploration period, mice were placed back in their homecage. After a 15- min retention interval, the mouse was placed in the apparatus for the second trial (choice phase), but now with a familiar one (a, sample) and a novel object (b). The objects were cleaned thoroughly with alcohol between sessions to remove any lingering scents. The time spent exploring each object during trial 1 and trial2 was recorded. Exploration was defined as either touching the object with the nose or being within 2 cm of it. The basic measures in the object recognition task were the times spent exploring an object during trial 1 and trial 2. Several variables were measured during the tests: e1 (a + a) and e2 (a + b) are measures of the total exploration time of both objects during trial 1 and trial 2, respectively. h1 is an index of habituation measured by the difference in total exploration time from trial 1 to trial 2(e1 - e2). d1 (b - a) and d2 (d1/e2) were considered as index measures of discrimination between the novel and the familiar objects. Thus, d2 is a relative measure of discrimination that corrects d1 for exploratory activity (e2). A discrimination index above zero describes animals exploring the novel object more than the familiar object. An animal with no preference for either object will have an index near zero. Mice with a total exploration time of less than 7 s during trials in the sample or choice phase were excluded from the analyses as the measurement of exploration time has been found to be non-reliable below this threshold(van den Buuse *et al. supra;* de Bruin et al, Pharmacol Biochem Behav 2006; 85(1):253-260).

**Elevated cross bar test.** The anxiety behaviour of mice based on their natural aversion of open and elevated areas was assessed using an elevated plus-maze as previously described (Komada et al. J Vis Exp 2008; (22); Walf et al. Nat Protoc 2007; 2(2):322-328). Briefly, the apparatus was made in the shape of a cross from black plexiglass and consisted of two open arms (25 cm x 5 cm) and two closed arms (25 cm x 5 cm x 16 cm) that crossed in the middle perpendicular to each other. In the middle of the to arms there was a central platform (5 cm x 5 cm). The cross maze was raised 1 m from the ground. Individual mice were introduced to the center of the apparatus facing the open arm opposite to the experimenter were and observed by video recording for 5 minutes. The number of entries into the open and closed arms and the time in exploring both types of arm were scored. Naturalistic behaviour of the mouse such as the number of head dipping, number of rearing and number of stretch attended postures were measured. After each trial all arms and the central area thoroughly cleaned with alcohol to remove any lingering scents.

**Escape water maze test.** Spatial learning and memory was assessed using a cross-maze escape task as previously described (Coyle *et al.* 2009, *supra).* The cross maze was made of a clear plastic (length, 72 cm; arm dimensions, length 26cm x width 20 cm) and placed in a circular pool of water (1 m diameter) maintained at 23 C. Milk powder was mixed into the water to conceal a submerged (0.5cm below the water surface) escape platform placed in the distal north arm of the maze. The pool was enclosed by a black plastic wall (height, 90 cm). Constant spatial cues were arranged at each arm of the maze and by the experimenter who always stood at the southern end during the training and testing procedures. 12 week old mice were individually habituated to the maze environment by being placed into the pool without the escape platform and allowed to swim for 60 s. Learning trials were conducted over a 6-day training period in which mice were required to learn the position of the submerged escape platform from the other three (East, South, West) arms that did not contain an escape platform. Each mouse was given six daily trials (two blocks of three trials separated by a 30min rest interval), in which each of the three arms were chosen as a starting point in a randomized pattern (twice daily). For each trial, the mouse was placed in the distal end of an arm facing the wall and allowed 60 s to reach the escape platform where it remained for 10 s. Mice that did not climb onto the escape platform in the given time were placed on the platform for 10 s. The mouse was then placed in a cage for 10 s and subsequent trials were continued. Mice were assessed on their long-term retention of the escape platform location which was placed in the same position as during the learning phase. Memory was tested 14 (M1 and 28 (M2) days after the final day of learning and consisted of a single day of 6 trials as described for the learning period. Data were recorded for each mouse for each trial on their escape latency (i.e. time (s) taken to swim to the platform), number of correct trials (i.e. if a mouse found the platform on the first arm entry) and number of incorrect entries/reentries (i.e. the number of times that a mouse went into an arm that did not contain the escape platform).

**PPI test.** Startle, startle habituation and PPI of startle were assessed using an eight-unit automated system (SR-LAB, San Diego Instruments, USA) as previously described (van den Buuse *et al.* 2009 *supra*). Briefly, mice were placed in clear Plexiglas cylinders which were closed on either side and acoustic stimuli were delivered over 70-dB background noise through a speaker in the ceiling of the box. Each testing session consisted of 104 trials with an average inter-trial interval between 25 s. The first and last eight trials consisted of single 40-ms 115 -dB pulse alone startle stimuli. The middle 88 trials consisted of pseudo-randomised delivery of 16 115-dB pulse-alone stimuli, eight trials during which no stimulus was delivered, and 64 prepulse trials. The total of 32 115-dB pulse alone trials was expressed as four blocks of eight and used to determine startle habituation. Prepulse trials consisted of a single 115-dB pulse preceded by a 30-ms or 100-ms inter-stimulus interval (ISI) with a 20-ms non-startling stimulus of 2, 4, 8 or 16 dB over the 70-dB baseline. Whole-body startle responses were converted into quantitative values by a piezo -electric accelerometer unit attached beneath the platform. Percentage prepulse inhibition (%PPI) was calculated as pulse-alone startle response - prepulse + pulse startle response / pulse-alone startle response X 100.

**Statistical analysis.** All statistical calculations are presented as mean ± SEM and were performed using SAS Version 9.2 (SAS Institute Inc.. Cary, NC, USA). For open field data the number of line crossings were compared across the WT and mutant groups and over time using a linear mixed effects model. A random mouse effect was included in the model to account for the dependence in repeated observations from the same mouse. Data from the elevated cross bar was compared between WT and mutants using an independent samples t-test. For the water cross-maze test escape latency was compared between the two treatment groups and over time using a Cox proportional hazards model. Robust variance estimation was used in the model to adjust for the dependence in results due to repeated measurements on the same mouse. In the model group (WT or KO), time (days I to 6) and the interaction between group and time were entered as predictor variables. Escape latency was considered right censored at 30 seconds when a mouse had yet to find the exit. In our study there were too many animals with an escape latency censored at 30 seconds to be able to treat the outcome as being normally distributed. Thus it was not feasible to use a linear mixed effects model. Incorrect entries were compared between WT and mutant groups and over time using a negative binomial regression model. In the model group (WT or KO), time (days 1 to 6) and the interaction between group and time were entered as predictor variables. A generalised estimating equation was used to account for the dependence in results due to repeated measurements on the same mouse. Data from the PPI tests were compared using two -way analysis of variance (ANOVA) with repeated measures (Systat, version 9.0, SPSS software: SPSS Inc., USA). For this analysis the between-group factor was genotype and the within group, repeated-measures factors were prepulse intensity and startle block. In all studies a*p* value of <0.05 was considered to be statistically significant.

**Immunohistochemistry.** Sections were blocked in 10% non-immune horse serum in PBST (0.1M PBS, 0.3% Triton X-100, 1% BSA) for 1h at room temperature (RT) and subsequently incubated with primary antibodies overnight at RT. Primary antibodies and dilutions: rabbit polyclonal to 14-3-3ζ (1:200) (Guthridge et al. Blood 2004; 103(3):820-827), rabbit polyclonal to 0-tubulin (1:250, Sigma), rabbit polyclonal to calbindin-D28K (1:1000. Chemicon), mouse monoclonal to NeuN (1:500, Chemicon), rabbit polyclonal to synaptophysin (1:100, Cell Signaling). On the following day, sections were incubated with secondary antibodies for 1 h at RT. After 3 times 0.1M PBS wash, the sections were mounted in Prolong® Gold antifade reagent with DAPI (Molecular Probes).

**BrdU-pulse-chase analysis and TUNEL labelling**. BrdU was injected at 100 µg / g of body weight of the pregnant mice at 14.5 dpc or 16.5 dpc and the pups were euthanized at postnatal-day-7. Final destination of the proliferating hippocampal neurons that were born at these time points were revealed by BrdU immunohistochemistry on frozen brain sections. Tissue were denatured with 2M HCI for 20 min at 37°C, neutralised in 0.1 M borate buffer (pH 8.5) for 10 min, blocked with 10% horse serum in PBST and probed with rat monoclonal anti-BrdU (1:250; Abcam) and mouse monoclonal anti-NeuN (1:500; Chemicon) antibodies overnight at 4°C. Cell apoptosis was determined by the TUNEL assay using the In Situ Cell Death Detection Kit (TMR Red; Roche Applied Science) according to the manufacturer's instructions followed by counterstained with DAPI (Molecular Probes).

**Immunoprecipitation.** All protein extracts were prepared by lysis in NP40lysis buffer composed of 150 mM NaCl, 10 mM Tris -HCl (pH 7.4), 10% glycerol, 1% Nonidet P-40, and protease and phosphatase inhibitors (10 mg of aprotinin per ml, 10 mg of leupeptin per ml, 2 mM phenylmethylsulfonyl fluoride, and 2 mM sodium vanadate). Samples were lysed for 60 min at 4 C, then centrifuged at 10,000g for 15 min. The supernatants were precleared with mouse 1g -coupled Sepharose beads for 30min at 4C. The precleared lysates were incubated for 2 h at 4C with 2 ug/ml of either anti-DISCI antibody (C-term) (Invitrogen) or anti-14-3-3 antibody (3F7 Abcam) absorbed to protein A-Sepharose (Amersham Biosciences). The sepharose beads were washed 3 times with lysis buffer before being boiled for 5 min in SDS-PAGE sample buffer. The immunoprecipitated proteins and lysates were separated by SDS-PAGE, and electrophorectically transferred to a nitrocellulose membrane and analysed by immunoblotting.

**Immunoblotting.** The membranes were probed with either anti-14-3-3ζ EB1 pAb at 1:1000 (Guthridge *et al.* 2004 *supra*) or anti DISC1 (C-term) (Invitrogen) at 1 ug/ml. ). For analysis of 14-3-3ζ from brain tissue rabbit polyclonal against the (3-actin (1:5000, Millipore) was used as a loading control. Bound antibodies were detected with HRP-conjugated secondary antibody (1:20,000, Pierce-Thermo Scientific). Immunoreactive proteins were visualized by ECL (Luminescent Image Analyzer LAS-4000, Fujifilm, Japan). The images were analysed with Multi Gauge Ver3.0 (Fujifilm, Japan).

Neuronal **cell cultures.** P7 hippocampi neuron-glial cocultures were prepared as described (Kaech et al. Nat Protoc 2006, 1(5):2406-2415). Nitric acid-treated coverslips (diameter 13 mm) were coated with 100 µg/ml poly-L-lysin/PLL (Sigma) in borate buffer for overnight at 37°C, and were then washed with sterile water for 3 x 1 h. Dentate gyri and CA samples were dissected and dissociated in Hank's balanced salt solution (HBSS) and neurons were plated at a density of I x 10⁵ cells per culture dish (with 4 PLL-coated coverslips). Cultures were incubated for 7 and 14 days *in vitro* for neurite outgrowth assay. Cells were fixed in 4% PFA for 1 h, preincubated in 10% non-immune horse serum in PBST (0.1 M PBS, 0.1% Triton X -100. 1% BSA) for 1 h at room temperature (RT) and incubated overnight at 4°C with primary antibodies against mouse monoclonal MAP2 (1:200, Millipore) and 14-3-3ζ (1:1000). The coverslips were then incubated with the corresponding secondary antibodies for 1 h at RT. Coverslips were mounted with anti -fade DAPI (Molecular Probes).

### Results

### 14-3-3ζ, mutant mice display behavioural and cognitive defects

14-3-3 proteins are abundantly expressed in the developing and adult brain (Berg et al. Nat Rev Neurosci 2003; 4(9):752-762; Baxter et al. Neuroscience 2002; 109(1):5-14). To ascertain the role of 14-3-3ζ in neurodevetopment and brain function generated two knockout mouse lines were generated from embryonic stem cell clones containing retroviral gene-trap insertions within intron 1 our 2, termed 14-3-3 and 14-3-3ζ^{Gt(OST390)Lex}, respectively (Fig. 8; Lexicon Genetics). Quantitative RT-PCR and western blot on embryonic and adult brain tissue from heterozygous intercrosses confirmed that the gene trap vectors disrupted gene transcription and created null alleles (Fig. 9). These mutant lines are referred to as 14-3-3ζ^{062+/.} and 14-3-3ζ^{390+/.}. Unlike deletions of other 14-3-3 isoforms (Su et al. Proc Natl Acad Sci USA 2011; 108(4):1555-1560), expression analysis further determined that removal of *14*-*3*-*3C* is not compensated by increased expression of other *14-3-3* family members in mutant mice (Fig. 10). Inter crosses of 14-3-3ζ heterozygous mice from both strains gave rise to homozygous mutants in the predicted Mendelian ratio (WT 23%, Het 56%, Mut 21%; n=494,*p*<0.001) indicating that removal of the gene is not embryonic lethal. Initial inspection of mutant embryos and newborn mice suggested that development proceeded normally as they were morphologically indistinguishable from their littermates. However, by P14 mutant mice from both lines showed growth retardation and by P21 around 20% of mutant mice had died (WT 29%, Het 54%, Mut 17%; n=1619). The remaining mutant mice were smaller than WT littermates but had similar life expectancy (P100; WT 24.55 ± 1.7g. Mut 19.73g ± 2.5g). Mutant mice appeared outwardly normal and healthy with no differences in the olfactory test, visual test and wire-hang test.

To definitively analyse the association of 14-3-3ζ with neurological disorders and brain functions, a series of behavioural tests on mutant and control mice were completed. The response of 14-3-3ζ^{062-/-} mice to an open field environment was first evaluated. Mutants showed a significant increase in distance travelled over the test period that was maintained throughout all testing ages (5, 10,20 and 30 weeks), indicating that mutant mice are hyperactive (Fig. 1A). This effect was similar for both males and females with no sex bias (*p>0.05*)*.*

The mouse's natural exploratory preference of novel objects rather than familiar objects was exploited to test recognition memory. Correct functioning of the perirhinal cortex in the medial lobe is essential for this task (Dere *et al.* 2006 *supra;* Sik *et al.* 2003 *supra;* Forwood et al. Hippocampus 2005; 15(3):347-355; Winters et al. J Neurosci 2005; 25(17):4243-4251). In the sample phase, mice spent an equal time exploring each identical object (14-3-3ζ^{062+/+}, 50.82±1.2%; 14-3-3ζ^{062-/-} 49.18±1.2%). When presented with a familiar and new object, 14-3-3ζ^{062-l-}mice exhibited signiticantly impaired novel object recognition compared to controls over the test period. Consistent with a lack of preference between the familiar and novel objects, 14-3-3ζ^{062-/-} mice had a reduced discrimination index (time exploring novel object - time exploring familiar object / time exploring novel object + time exploring familiar object) indicating that they failed to retain new information (14-3-3ζ^{062+/+}, 0.1667±0.086s; 14-3-3ζ^{062-/-}, -0.0569±0.047s; *p*<0.05). Once again, there were no sex differences in either phase of testing (*p*>0.5). Notably, 14-3-3ζ^{062-/-} mutants also demonstrated hyperactivity in the object recognition test with longer exploratory times in both phases of the trial (Sample phase, 14-3-3ζ0^{62+/+}, 27.33±2.7s; 14-3-3ζ^{062-/-}, 38.62±4.1s; *p<0,05*; test phase, 14-3-3ζ^{062+/+},24.58±3.1s; 14-3-3ζ^{062-/-}, 50.77±4.7s;*p*<0.0001).

The elevated plus maze is widely used to test anxiety behaviour of rodents(Komada *et al.* 2008 *supra;* Walf *et al.* 2007 *supra;* Lister RG, Psychopharmacology (Berl) 1987; 92(2):180-185). When placed in such a test, 14-3-3ζ^{062-/-} mice also demonstrated increased activity compared to wild type controls. 14-3-3ζ^{062-/-} mice had 25.23 ± 1.76 transitions between cross arms during a 5min test period while 14-3-3ζ^{062+/+} had 12.29 ± 1.21 (*p*<0.0001). In addition, 14-3-3ζ^{062-/-} mice spent significantly more time in the open arms (Fig. 1B: 114.8 ± 11.5s) compared to 14-3-3ζ^{062+/+} mice (31.4 ± 6.0s , *p*<0.0001), entered them more often (14-3-3ζ^{062+/+}, 4.6 ± 0,6 ; 14-3-3ζ^{062-/-}, 15.5 ± 1.7. *p*<0.0001) and head dipped more, 14-3-3ζ^{062+/+} 19.6 ± 1.5; 14-3-3ζ^{062-/-.}33.4 ± 2.4 *p=* 0.0041) suggesting that they had lower levels of anxiety.

Spatial working memory-dependent learning was examined using a cross maze escape task (Summers *et al.* 2006 *supra*). Appropriate signalling between the hippocampus and prefrontal cortex are a prerequisite for acquisition of this task. Mice were trained over 6 days to identify the correct arm of a cross maze containing a submerged escape platform. Each arm of the cross maze was denoted by a novel visual cue throughout the experiment. Although some 14-3-3ζ^{062-/-} mice learnt to identify the correct arm. they showed increased latency in reaching the platform over the course of the acquisition period (Fig. 11; χ² (5) = 29.8808; p< 0.0001) and had significantly decreased arm choice accuracy (Fig. 1 C: IRR = 0.52; *p<* 0.0001). Their ability to remember the correct cross-arm was then tested by resting them for 14 days or 28 days post acquisition followed by re-testing in the escape platform water maze (M1 and M2, respectively). In comparison to the learning phase, 14-3-3ζ^{62+/+}mice showed no change in escape latency (HR =1.18,*p* = 0.383), whilst 14-14-3-3ζ^{062-/-}demonstrated significantly increased escape latency (HR = 2.98, *p* < 0.0001). Consistent with dysfunction in hippocampus-dependent memory, mutant mice also had a significant decrease in arm choice accuracy (Fig. 1C: IRR = 0.231; *p<* 0.0001). All cognitive defects were independent of sex.

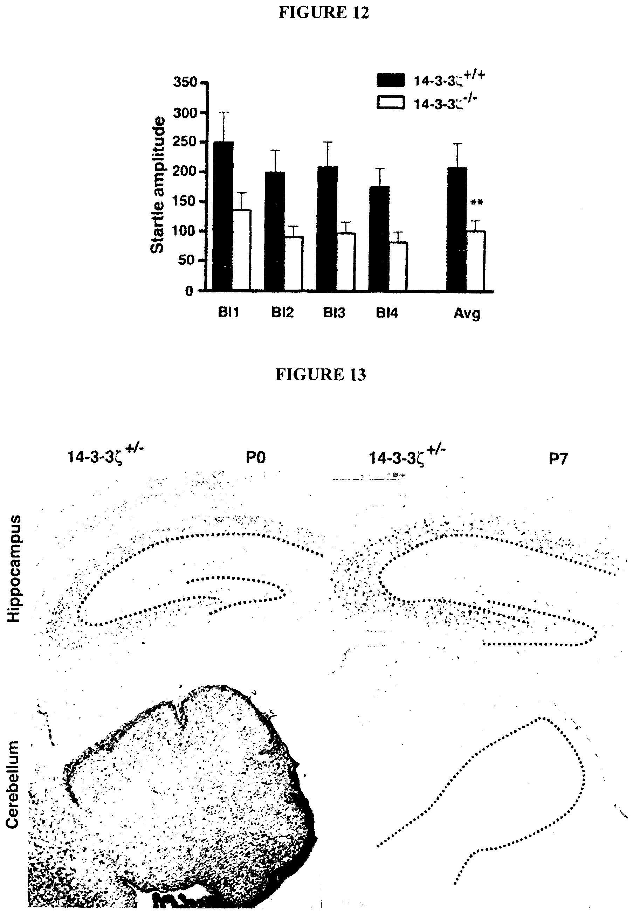

Defects in sensorimotor gating are an endophenotype of neuropsychiatric disorders such as schizophrenia and related disorders. Appropriate signalling in the hippocampus and other brain regions are essential for this filtering mechanism. To determine if 14-3-3ζ mutant mice have abnormal sensorimotor gating, prepulse inhibition (PPI) of the acoustic startle reflex was assessed. It was found that 14-3-3^{062-/-} mice had a significantly lower PPI (Fig. ID: main effect of genotype F(1,20) = 5.89, *p =* 0.025) and startle (Fig. 12: F(1,20) = 5.87, p= 0.023) compared to 14-3-3ζ^{062-/-} mice. Increasing levels of prepulse intensities caused similar increases in PPI in WT and mutant mice (Fig. ID). Overall, startle amplitudes were reduced in mutant mice but startle habituation was normal (Fig. 12).

### 14-.3-3ζ, is expressed in hippocampal neurons to control lamination

To determine if the cognitive and behavioural deficits arise from neurodevelopmental defects of the hippocampus, the role of 14-3-3ζ in neuronal development was analysed. Hippocampal neurons derive from the neuroepithelium along the ventricular zone (NEv) and from a restricted area of neuroepithelium adjacent to the fimbria (NEf) (Nakahira et al. J Comp Neurol 2005; 483(3):329-340) (Fig. 2A). At 14.5 dpc 14-3-3ζ immunostaining was detected in migrating hippocampal neurons within the intermediate zone, but not in their neuroepithelial precursors (Fig. 2Bi). By P0 14-3-3ζ immunostaining was also detected in pyramidal cells of the hippocampal proper/cornu ammonis (CA) (Fig. 2Biii). Taking advantage of the Beta-geo transgene within the gene trap vectors of the 14-3-3ζ mouse lines endogenous expression of *14*-*3-3ζ* with B-galactosidase staining in heterozygous mice was monitored. Consistent with immunostaining, expression of *14-3-3ζ at* the transcript level in migrating CA neurons was identified. In addition, expression within CA and DG neurons was detected into late adulthood (Fig. 2C). Unexpectedly, however. 14-3-3ζ was undetectable in other regions of brain, such as the cerebellum, after early post natal stages (Fig. 13). Expression within CA and DG neurons was confirmed by western blot of protein extracted from microdissected adult hippocampi (Fig. 2D). This also confirmed complete removal of the protein from these brain regions of 14-3-3ζ062-/- mice. Finally, after 10 days in vitro (DIV), hippocampal MAP2 positive neuronal cultures also showed punctate immunocytostaining for 14-3-3ζ within the cell body and axon / dendrites (Fig. 2E).

As 14-3-3ζ is expressed in hippocampal neurons we next examined if CA and DG neurons were examined to determine if they are positioned correctly in adult and embryonic mutants. Nissl-staining of 14-3-3ζ^{062-/-} mice revealed developmental defects first noticeable prior to hippocampal maturation (5/5 at P0, 4/4 at P7, 2/2 at P28 and 2/2 at P56; Fig. 3A and Fig. 14). Specifically, pyramidal neurons were ectopically positioned in the stratum radiatum and stratum oriens in addition to their usual resting place of the stratum pyramidale. Within the CA3 subfield, pyramidal neurons split in to a bilaminar stratum instead of a single cell layer.. Dentate granule neurons were also diffusely packed in the 14-3-3ζ^{062-/-} mice compared with 14-3-3ζ^{062+/+} littermates. Consistent with Nissl staining, analysis of hippocampal organization in *thyl* -YFP mice also revealed a disrupted laminar organization (Fig. 3B).

Consideration was then directed to whether ectopically positioned pyramidal cells developed into mature neurons. In all 14-3-3ζ^{062-/-} hippocampi (4/4 pups) ectopic cells were positive for the neuronal marker NeuN (Fig. 3C). Rather than positioning themselves in the deep molecular layer, neurons also matured in the superficial layer of CA3. Together, this data infers that mispositioned cells in the hippocampus form functional pyramidal and granular neurons. Additionally, TUNEL staining of hippocampi from embryonic, early postnatal and adult mice showed no apparent differences between genotypes (Fig. 15) suggesting that lack of 14-3-3ζ does not affect neuronal viability.

### 14-3-3ζ-deficient mice display hippocampal neuronal migration defects

The expression of 14-3-3ζ within the intermediate zone at 14.5dpc and the presence of mature neurons in the superficial layer at P0 raised the possibility that the aberrant laminar structure may arise from erroneous migration. To visualize hippocampal neuron migration, BrdU birthdating was completed by injecting BrdU into pregnant dams from heterozygous 14-3-3ζ⁰⁶² crosses at 14.5 dpc and 16.5 dpc. 14-3-3ζ^{062+/+} and 14-3-3ζ^{062-/-} pups were collected at P7 and BrdU-retaining cells were identified in coronal sections. Sections were counterstained with DAPI to identify separate layers of the hippocampus. BrdU-retaining cells were counted from 10µm sections using 5 mice of each genotype and the relative percentage in each layer was quantified. Both injection time points show that nearly all neurons born in the ventricular zone at 14.5 dpc or 16.5 dpc migrate in to the stratum pyramidalc of the CA in control mice (Fig. 4). Strikingly, however, a significant percentage of BrdU-retaining cells were identified outside of the stratum pyramidale in 14-14-3-3ζ^{062-/-} mice. Failure of neurons to migrate from their birthplace or to stop within their correct layer therefore gives rise to the duplicated stratum pyramidale in the 14-3-3ζ^{062-/-} hippocampus.

### Functional disrupted mossyfibre circuit and aberrant synaptic terminals in pyramidal cells in 14-3-3ζ-deficient mice

Communication between the CA3 pyramidal neurons and DG granule cells is achieved through precise axonal navigation and synaptic targeting. The issue of whether misaligned pyramidal neurons affected the hippocampal circuit was assessed by performing immunohistochemical staining with anti-calbindin in P0, P7 and P56 hippocampi. In control mice, mossy fibres sprouted from the somata of the granule cells and bifurcated into infrapyramidal mossy fibre (IPMF) and suprapyramidal mossy fibre (SPMF) tracts spanning the stratum pyramidale of CA3 (Fig. 5). In 14-3-3ζ^{062-/-} mice the IPMF tract navigated along the apical surface of CA3 pyramidal neurons, however, the SPMF tract was misrouted amongst the CA3 neurons.

To determine whether DG granular cells synapsed on their CA target cells, anti-synaptophysin was used to identify presynapses in both the IPMF and SPMF of the CA3 subfield in control animals. In 14-3-3ζ^{062-/-} mice, misrouted axons also formed aberrant synapses within the stratum pyramidale (Fig. 6). Visualisation of synaptic boutons by golgi stain further revealed notable differences in synapse formation in CA3. In control animals large spine excrescences on the proximal region of the apical dendrites were followed by fine-calibre dendritic branches. In pyramidal neurons of 14-3-3ζ^{062-/-} mice the dendritic tree appeared to have similar numbers of branch points but had thorny excrescences from the misrouted mossy fibre tracts on both proximal and distal apical dendrites of all mice examined.

To identify the molecular pathways employed by 14-3-3ζ to coordinate neuronal migration and axonal pathfinding co-immunoprecipitation experiments were performed on whole brain extracts from P7 mice. It was found that 14-3-3ζ could be co-immunoprecipitated with an antibody raised to the C-terminus of DISC1. Vice versa, it was also found that DISC1 could be co-immunoprecipitated with an antibody recognising 14-3-3ζ (Fig. 7). Surprisingly, the data indicate that 14-3-3ζ interacts specifically with the 75 kDa form of DISC1 rather than the 100 kDa full length protein, indicating that DISC1 functions in an isoform specific manner in neurodevelopment.

### EXAMPLE 2

### DEMONSTRATION THAT NRP2 POSITIVE NEURONAL PRECURSORS GIVE RISE TO THE HIPPOCAMPUS

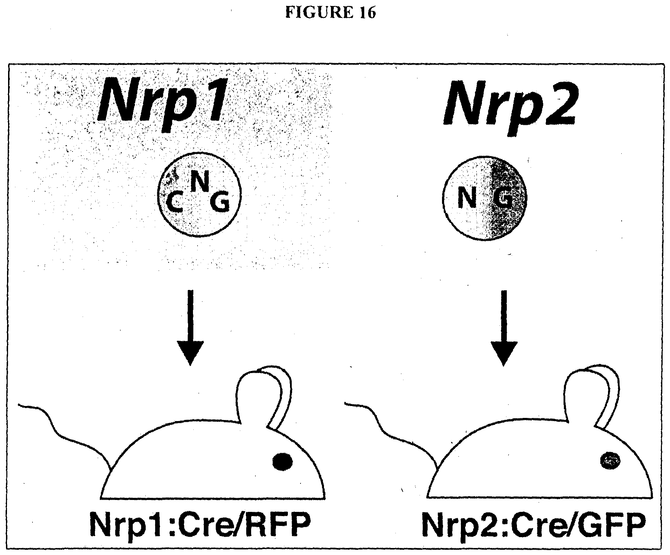

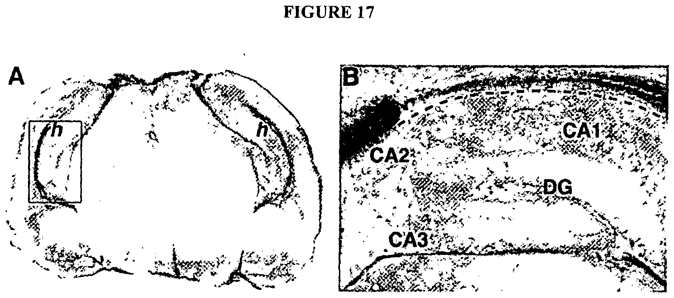

In order to determine the mature neurons that derive from the neuronal precursors expressing Nrp1 or Nrp2, Nrp2 and Nrp2 lineage tracing mice have been generated. For this Cre/RFP or Cre/GFP have been placed under the expression of the Nrp1 or Nrp2 promoters (Figure 16). Studies with these mice (from n=2 experiments Figure 17) show for the first time that neurons of the hippocampus are derived from Nrp2-expressing neural stem cells.

### BIBLIOGRAPHY

Aitken A., Semin Cancer Biol 2006; 16(3):162-172
Amaral and Lavenex (2006). "Ch 3. Hippocampal Neuroanatomy". In Andersen et al. The Hippocampus Book. Oxford University Press
Baxter et al. Neuroscience 2002; 109(1):5-14
Berg et al. Nat Rev Neurosci 2003; 4(9):752-762
Coyle et al. Behav Brain Res 2009, 197(1): 210-218
de Bruin et al. Pharmacol Biochem Behav 2006; 85(1):253-260
Dere et al. Neurosci Biohehav Rev 2006; 30(8):1206-1224
Eichenbaum et al. (2007), Annu Rev Neurosci 30:123-52
Forwood et al. Hippocampus 2005; 15(3):347-355
Fu et al. Annu Rev Pharmacol Toxicol 2000; 40:617-647
Guthridge et al. Blood 2004; 103(3):820-827
Kaech et al. Nat Protoc 2006, 1(5):2406-2415
Komada et al. J Vis Exp 2008; (22)
Lister RG, Psychopharmacology (Berl) 1987; 92(2):180-185
Middleton et al. Neuropsychopharmacoloy 2005; 30(5):974-983
Moser and Moser (1998) Hippocampus 8(6): 608-19
Nakahira et al. J Comp Neurol 2005; 483(3):329-340
Rosner et al. Amino Acids 2006: 30(1):105-109
Sik et al. Behav Brain Res 2003; 147(1-2):49-54
Su et al. Proc Natl Acad Sci USA 2011; 108(4):1555-1560
Summers et al. Pediatr Res 2006; 59(1): 66-71
Toyo-oka et al. Nat Genet 2003 Jul; 34(3): 274-285
van den Buuse et al. Int J Neuropsychopharmacol 2009; 12(10):1333-1393
Walf et al. Nat Protoc 2007; 2(2):322-328
Winson (1978), Science 201(4351):160-63
Winters et al. J Neurosci 2005; 25(17):4243-4251
Wong et al. Schizophr Res 2005; 78(2-3):137-146
Xu, et al., Nat Biotechnol. 2001 Oct; 19(10):971-4

## Claims

1. An Nrp2⁺ neural crest stem cell population or variants thereof for use in the treatment of a neuropsychiatric condition in a mammal, which condition is **characterised by** a defective hippocampus, wherein said stem cells or variants thereof regenerate the hippocampus.

2. The cell population for use according to claim 1 wherein said Nrp2⁺ neural crest stem cells are adult stem cells.

3. The cell population for use according to claim 2 wherein said adult Nrp2⁺ neural crest stem cells are isolated from the dentine of teeth or hair follicles.

4. The cell population for use according to any one of claims 1-3 wherein said condition is **characterised by** a reduction in the level of functional protein 14-3-3ζ or protein 14-3-3ζ/DISC1 complex formation.

5. The cell population for use according to claim 4 wherein said neuropsychiatric condition is a condition **characterised by** one or more symptoms of schizophrenia, schizophrenia, schizotypal personality disorder, psychosis, bipolar disorder, manic depression, affective disorder, or schizophreniform or schizoaffective disorders, psychotic depression, autism, drug induced psychosis, delirium, alcohol withdrawal syndrome or dementia induced psychosis.

6. The cell population for use according to any one of claims 1-5 wherein said mammal is a human.

## Patentansprüche

1. Nrp2⁺-Neuralleistenstammzellpopulation oder Varianten davon zur Verwendung bei der Behandlung eines neuropsychiatrischen Leidens bei einem Säugetier, wobei das Leiden durch einen defektiven Hippocampus gekennzeichnet ist, wobei die Stammzellen oder Varianten davon den Hippocampus regenerieren.

2. Zellpopulation zur Verwendung nach Anspruch 1, wobei es sich bei den Nrp2⁺-Neuralleistenstammzellen um adulte Stammzellen handelt.

3. Zellpopulation zur Verwendung nach Anspruch 2, wobei die adulten Nrp2⁺-Neuralleistenstammzellen aus dem Dentin von Zähnen oder Haarfollikeln isoliert werden.

4. Zellpopulation zur Verwendung nach einem der Ansprüche 1-3, wobei das Leiden durch eine Reduktion der Konzentration des funktionellen Proteins 14-3-3ζ oder der Protein-14-3-ζ/DISC1-Komplexbildung gekennzeichnet ist.

5. Zellpopulation zur Verwendung nach Anspruch 4, wobei es sich bei dem neuropsychiatrischen Leiden um ein durch ein oder mehrere Symptome von Schizophrenie gekennzeichnetes Leiden, Schizophrenie, eine schizotypische Persönlichkeitsstörung, Psychose, bipolare Störung, manische Depression, eine affektive Störung oder schizophreniforme oder schizoaffektive Störungen, psychotische Depression, Autismus, drogen-oder arzneimittelinduzierte Psychose, Delirium, Alkoholentzugssyndrom oder demenzinduzierte Psychose handelt.

6. Zellpopulation zur Verwendung nach einem der Ansprüche 1-5, wobei es sich bei dem Säugetier um einen Menschen handelt.

## Revendications

1. Population de cellules souches de la crête neurale Nrp2⁺ ou variantes de celles-ci pour utilisation dans le traitement d'un état neuropsychiatrique chez un mammifère, ledit état étant **caractérisé par** un hippocampe défectueux, où lesdites cellules souches ou variantes de celles-ci régénèrent l'hippocampe.

2. Population de cellules pour utilisation selon la revendication 1, où lesdites cellules souches de crête neurale Nrp2⁺ sont des cellules souches adultes.

3. Population de cellules pour utilisation selon la revendication 2, où lesdites cellules souches de crête neurale Nrp2⁺ adultes sont isolées d'avec la dentine des dents ou des follicules capillaires.

4. Population de cellules pour utilisation selon l'une quelconque des revendications 1 à 3, où ledit état est **caractérisé par** une réduction du niveau de la protéine fonctionnelle 14-3-3ζ, ou la formation du complexe protéine 14-3-3ζ/DISC1.

5. Population de cellules pour utilisation selon la revendication 4, où ledit état neuropsychiatrique est un état **caractérisé par** un ou plusieurs symptômes choisis parmi les suivants : schizophrénie, schizophrénie, trouble de personnalité schizotypique, psychose, trouble bipolaire, trouble maniaco-dépressif, trouble affectif, ou troubles schizophréniformes ou schizo-affectifs, dépression psychotique, autisme, psychose induite par médicament, délire, syndrome de manque d'alcool ou psychose induite par la démence.

6. Population de cellules pour utilisation selon l'une quelconque des revendications 1 à 5, où ledit mammifère est un humain.
